# EUROPÄISCHE PATENTSCHRIFT

(11) **EP 0 393 424 B1**
(45) Veröffentlichungstag und Bekanntmachung des Hinweises auf die Patenterteilung: **27.09.1995**
(21) Anmeldenummer: 90106459.2
(22) Anmeldetag: 04.04.1990
(51) Int. Cl.: C07D 487/04, C07D 487/14, C07D 471/14, C07D 513/14, C07C 271/18

(54) **Verfahren zur Herstellung von 2,7-Diazabicyclo(3.3.0)octanen**
Process for the preparation of 2,7-Diazabicyclo(3.3.O)octanes
Procédé de préparation de 2,7-Diazabicyclo(3.3.O)octanes

(30) Priorität: 17.04.1989 DE 3912509; 03.10.1989 DE 3932903
(43) Veröffentlichungstag der Anmeldung: 24.10.1990
(73) Patentinhaber: BAYER AG, 51368 Leverkusen (DE)
(72) Erfinder: Schenke, Thomas, Dr., D-5060 Bergisch-Gladbach (DE); Petersen, Uwe, Dr., D-5090 Leverkusen (DE)

(56) Entgegenhaltungen:
- EP-A- 0 169 169
- EP-A- 0 350 733
- EP-A- 0 391 132

## Beschreibung

Die vorliegende Erfindung betrifft Verfahren zur Herstellung von 2,7-Diazabicyclo[3.3.0]octanen. Diese Verbindungen sind wertvolle Zwischenprodukte zur Herstellung hochwirksamer antibakterieller Chinoloncarbonsäuren.

Aus der EP-A-0 350 733 und der EP-A-0 391 132 sind bereits 2,7-Diazabicyclo[3.3.0]octane sowie durch sie erhältliche Chinoloncarbonsäurederivate bekanntgeworden.

Es ist bereits bekanntgeworden, daß 2,7-Diazabicyclo[3.3.0]octan (Oktahydropyrrolo[3,4-b]pyrrol) durch Reduktion mit Lithiumaluminiumhydrid aus 2,7-Diazabicyclo[3.3.0]octan-3,8-dion darstellbar ist. Diese Verbindung wurde durch Addition von Diazomethan an Glutaconsäuredimethylester und anschließende hydrierende Spaltung des intermediären Pyrazolinderivates hergestellt (Justus Liebigs Ann. Chemie 677, 154 (1964)). Dieses Verfahren birgt den Nachteil des Umgangs mit dem hochtoxischen und explosiven Diazomethan in sich und ist für eine technische Anwendung unbrauchbar. Darüberhinaus sind substituierte Glutaconsäureester nur schwierig darstellbar, so daß dieses Verfahren nicht ohne weiteres verallgemeinerungsfähig ist.

Die Erfindung betrifft Verfahren zur Herstellung von 2,7-Diazabicyclo[3.3.0]octanen der Formel (I), 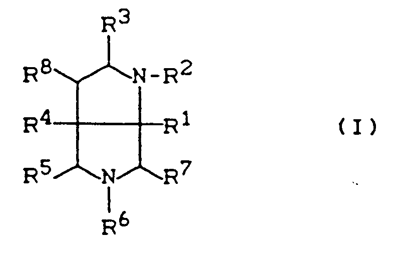
wobei
- R¹, R³, R⁴, R⁵, R⁷, R⁸: gleich oder verschieden sein können und jeweils H, C₁-C₅-Alkyl, gegebenenfalls substituiert mit Halogen, Hydroxy oder C₁-C₄-Alkoxy, C₁-C₃-Alkoxycarbonyl, C₆-C₁₂-Aryl, bevorzugt H, C₁-C₃-Alkyl, C₁-C₃-Alkoxycarbonyl, Phenyl, besonders bevorzugt H oder Methyl bedeuten,
- R⁴: zusätzlich Halogen, bevorzugt Fluor, Chlor oder Brom, besonders bevorzugt Chlor oder Brom bedeutet,
- R² und R⁶: gleich oder verschieden sein können und H, C₁-C₆-Alkyl, Benzyl, C₆-C₁₂-Aryl, C₁-C₃-Alkanoyl, Benzoyl, C₁-C₅-Alkoxycarbonyl, bevorzugt H, C₁-C₃-Alkyl, Benzyl, C₆-C₁₂-Aryl, C₁-C₂-Alkanoyl, Benzoyl, C₁-C₄-Alkoxycarbonyl, besonders bevorzugt H, Methyl, Phenyl, Acetyl, C₂-C₄-Alkoxycarbonyl bedeuten und
- R² und R³: zusammen gegebenenfalls eine Brücke der Struktur (CH₂)ₙ, n = 2 - 4, CH₂-CHOH-CH₂, CH₂-S-CH₂, C(CH₃)₂-S-CH₂ bedeuten.

Besonders bevorzugt hergestellt werden die Verbindungen der folgenden Formeln: 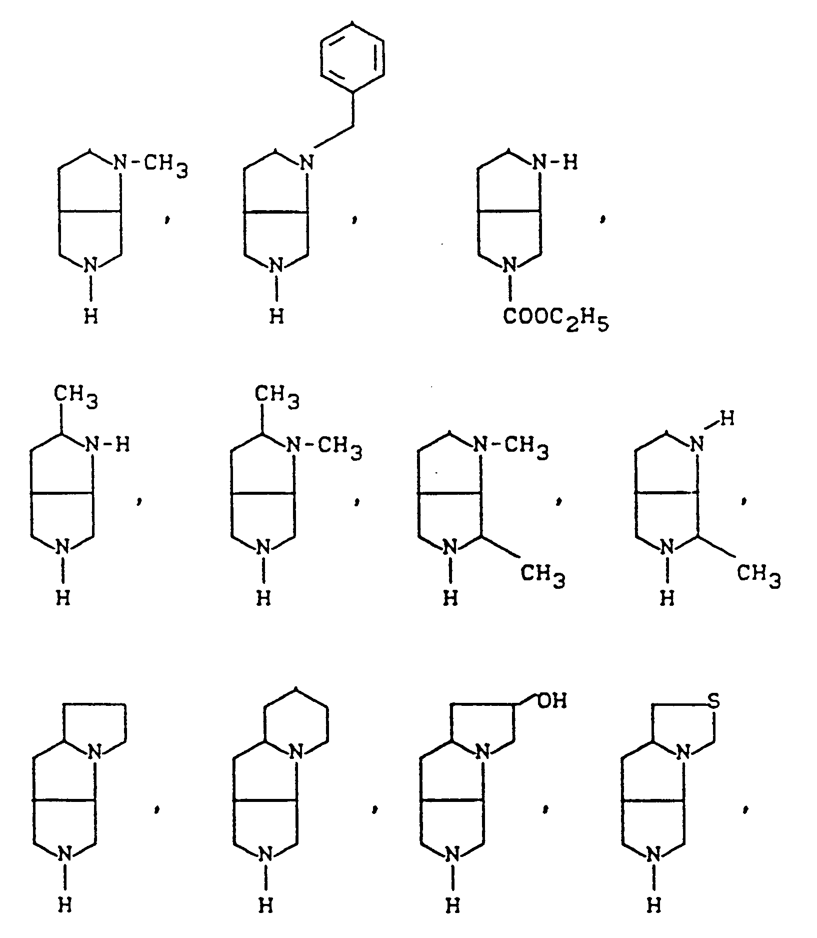 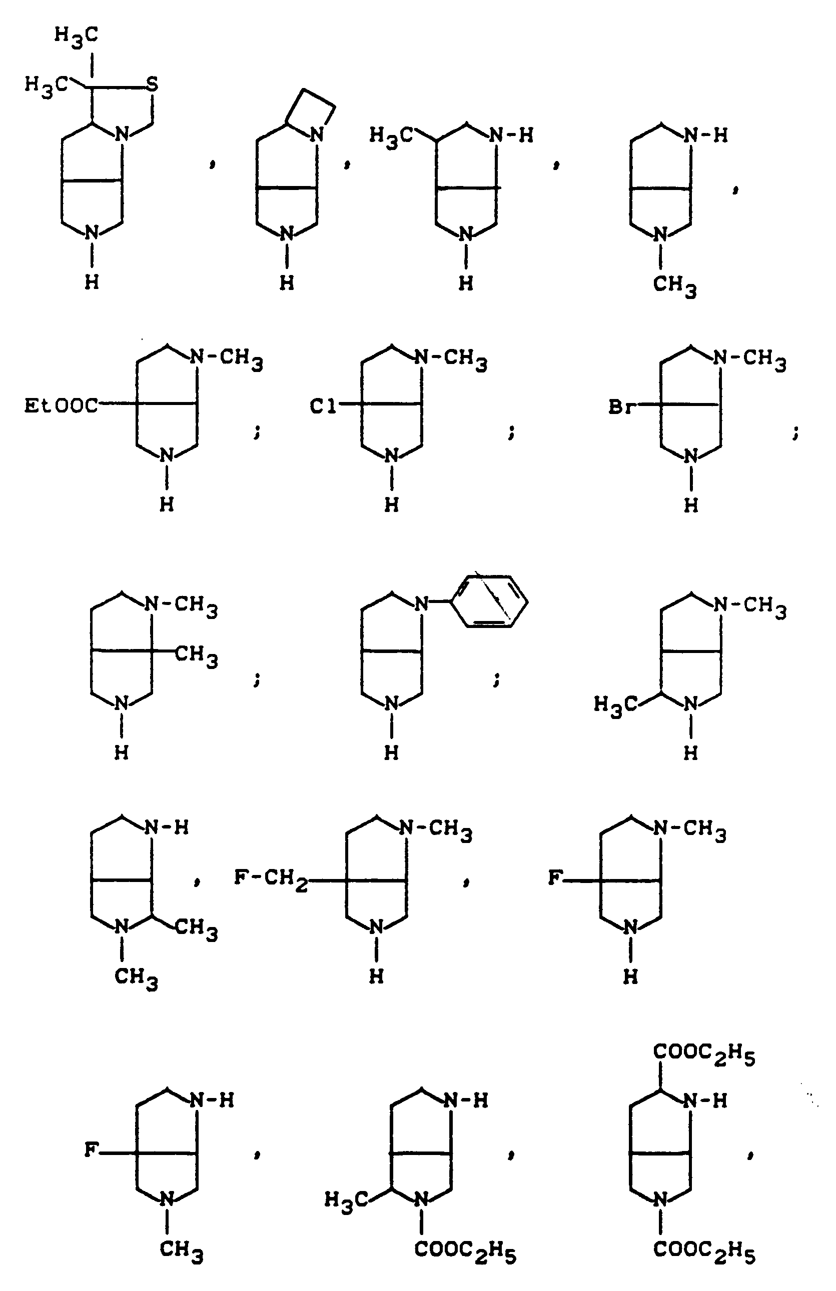
Der C₆-C₁₂-Arylrest kann substituiert oder unsubstituiert sein. Als Substituenten kommen 1 bis 3, bevorzugt 1 Substituent(en) aus der Gruppe Halogen, insbesondere Cl, Br oder F, Amino, C₁-C₃-Alkylamino, C₁-C₃-Dialkylamino, Hydroxy, C₁-C₃-Alkoxy, C₁-C₃-Alkyl oder Cyano in Frage.

Es wurde weiterhin gefunden, daß man 2,7-Diazabicyclo[3.3.0]octane der Formel (I) 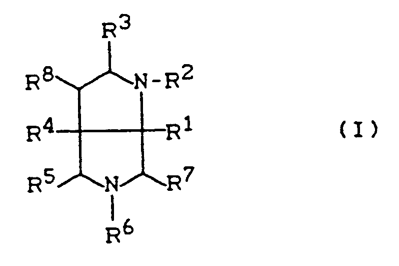
wobei
- R¹, R³, R⁴, R⁵, R⁷, R⁸: gleich oder verschieden sein können und jeweils H, C₁-C₅-Alkyl, gegebenenfalls substituiert mit Halogen, Hydroxy, C₁-C₃-Alkoxy, C₁-C₃-Alkoxycarbonyl, C₆-C₁₂-Aryl, bevorzugt H, C₁-C₃-Alkyl, C₁-C₃-Alkoxycarbonyl, Phenyl, besonders bevorzugt H und Methyl bedeuten,
- R⁴: zusätzlich Halogen, bevorzugt Fluor, Chlor oder Brom, besonders bevorzugt Chlor oder Brom bedeutet,
- R² und R⁶: gleich oder verschieden sein können und H, C₁-C₆-Alkyl, Benzyl, C₆-C₁₂-Aryl, C₁-C₃-Alkanoyl, Benzoyl, C₁-C₅-Alkoxycarbonyl, bevorzugt H, C₁-C₃-Alkyl, Benzyl, C₆-C₁₂-Aryl, C₁-C₂-Alkanoyl, Benzoyl, C₁-C₄-Alkoxycarbonyl, besonders bevorzugt H, Methyl, Phenyl, Acetyl, C₂-C₄-Alkoxycarbonyl bedeuten und
- R² und R³: zusammen gegebenenfalls eine Brücke der Struktur (CH₂)ₙ, n = 2 - 4, CH₂-CHOH-CH₂, CH₂-S-CH₂, C(CH₃)₂-S-CH₂ bedeuten,
dadurch erhält, daß man ungesättigte Carbonylverbindungen der Formel (II) mit Aminosäurederivaten der Formel (III) in einer intramolekularen 1,3-dipolaren Cycloaddition umsetzt, 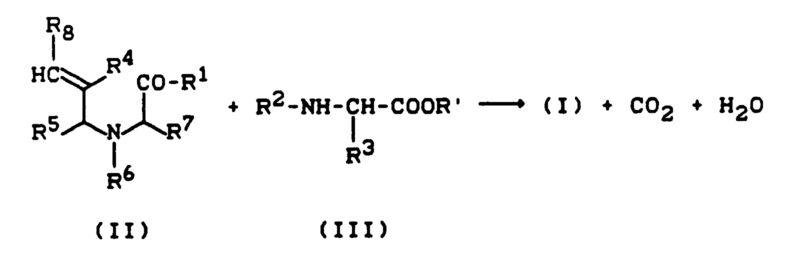
worin
R′ für H oder C₁-C₃-Alkyl steht und
wobei R¹ - R⁸ die oben angegebene Bedeutung haben. Anschließend können die Substituenten R² oder R⁶, welche eine Schutzgruppenfunktion haben, abgespalten werden.

Der Vorteil des erfindungsgemäßen Verfahrens besteht in der Einfachheit seiner Durchführung und in der leichten Zugänglichkeit der Ausgangsverbindungen (II) und (III). Als besonders vorteilhaft muß die hohe Stereoselektivität des Verfahrens bezeichnet werden. Dies ist besonders überraschend, da ähnliche Cyclisierungen zu Produktgemischen führen können (J. Chem. Soc., Chem. Comm. 1984, 182). Durch die Selektivität des hier beanspruchten Verfahrens entfällt eine verlustreiche und unökonomische Abtrennung unerwünschter Diastereomerer.

Der Reaktionsverlauf des erfindungsgemaßen Verfahrens sei durch folgende Beispiele erläutert: 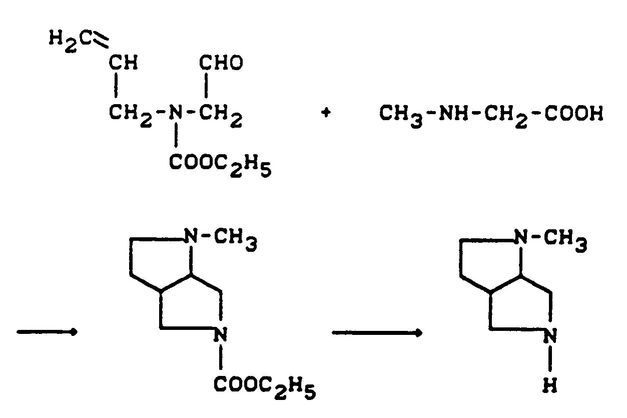 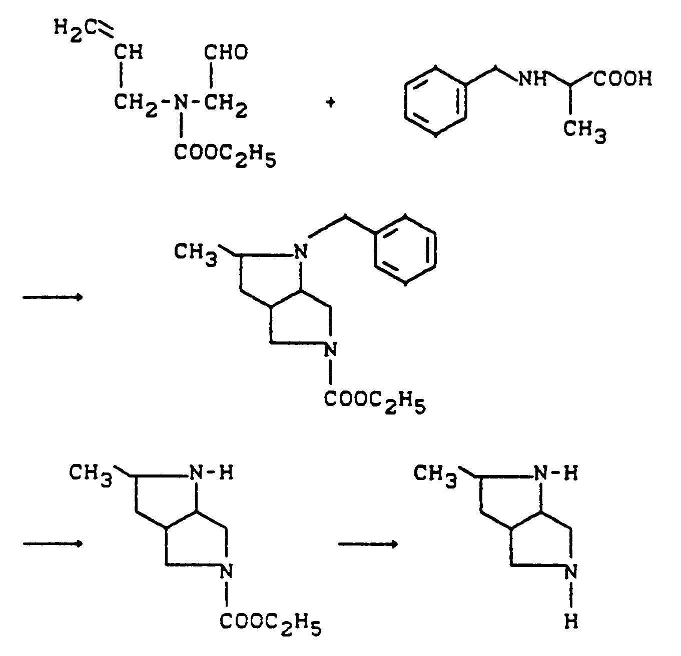
Die als Ausgangsverbindungen benötigten ungesättigten Carbonylverbindungen der Formel (II) sind neu. Sie können nach folgenden Verfahren hergestellt werden:
1. Ausgehend von käuflichem Aminoacetaldehyddimethylacetal wird die Aminogruppe acyliert, das Amid in Gegenwart von starken Basen mit Allylhalogeniden alkyliert und das Acetal sauer gespalten. 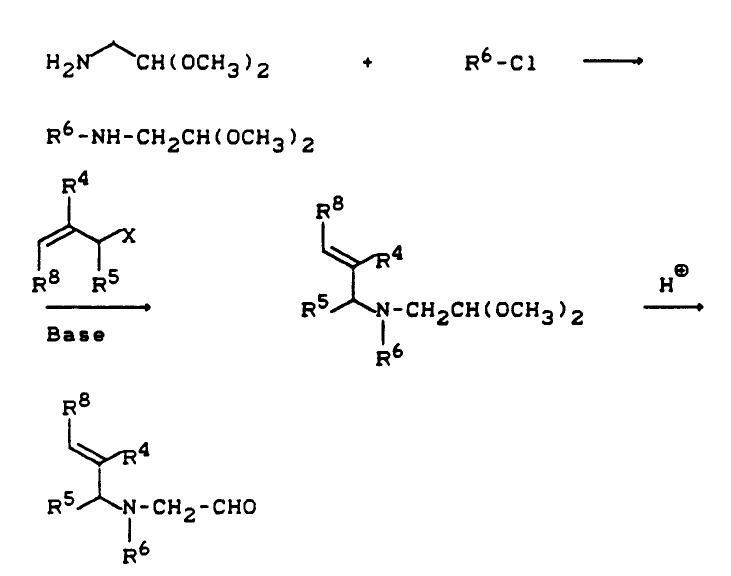 Das bekannte Acetamidoacetondimethylacetal (Synthesis 1988, 381) kann in Gegenwart starker Basen mit Allylhalogeniden alkyliert und die Acetalgruppe sauer verseift werden. 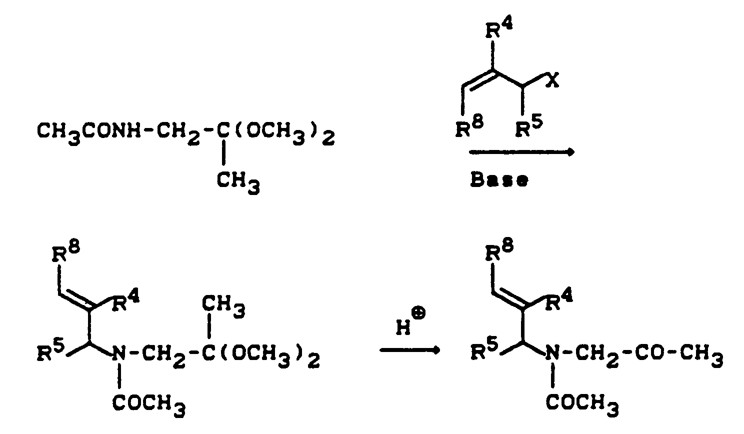
2. Geht man von käuflichen oder bekannten (EP-A-0 249 530, 03.06.1986) α-Ketoaldehydmonoacetalen aus, kann man diese mit Allylaminen reduktiv aminieren. Nach Einführen einer (Schutz-)gruppe R⁶ durch Alkylieren oder Acylieren wird die Acetalgruppe sauer verseift. 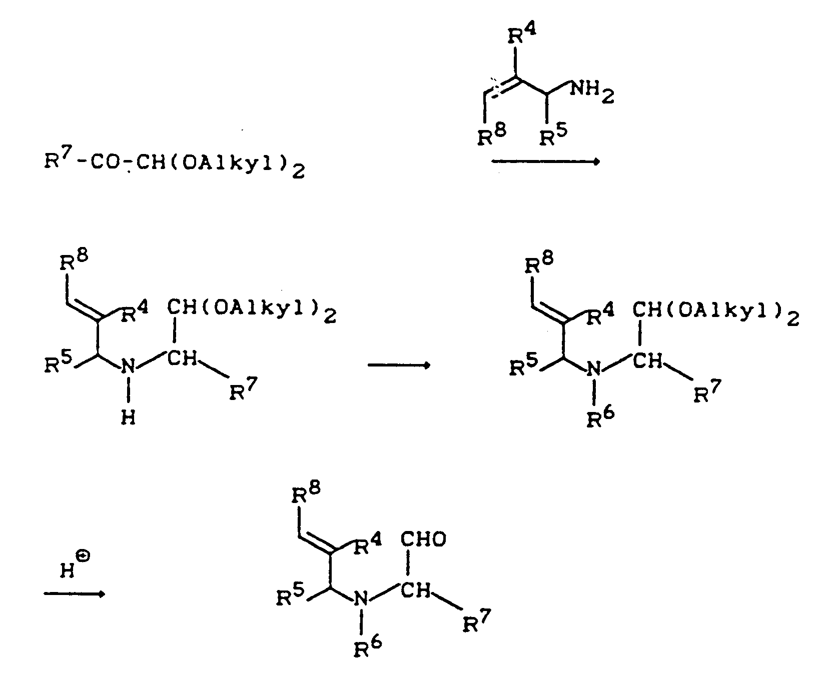
3. Ungesättigte Aldehyde oder Ketone können mit Aminoacetaldehyddimethylacetal reduktiv aminiert werden. Nach Einführen der Gruppe R⁶ durch Alkylieren oder Acylieren kann die Acetalgruppe sauer gespalten werden. 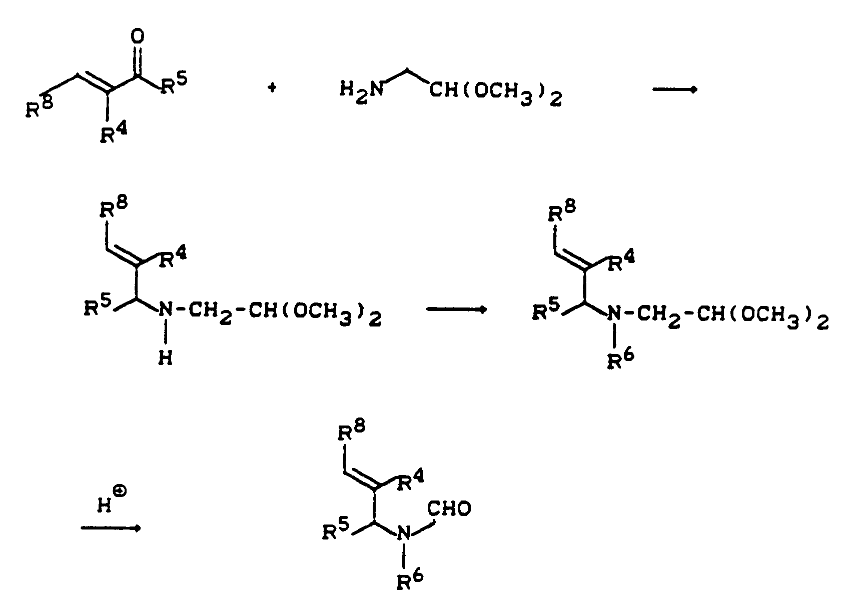
4. N-Allylaminoalkohole erhält man
   a) durch Ringöffnung von Epoxiden mit Allylaminen (J. Pharm. Soc. Japan 73, 1330 (1953)),
   b) durch Alkylieren von substituierten Ethanolaminen mit Allylhalogeniden (J. Am. Chem. Soc. 64, 1692 (1942); 72, 3536 (1950)),
   c) durch reduktive Aminierung von ungesättigten Aldehyden oder Ketonen mit substituierten Ethanolaminen.

   Nach Einführung der Gruppe R⁶ durch Alkylieren oder Acylieren wird die Alkoholfunktion mit geeigneten Oxidationsmitteln zu Verbindungen der Formel (II) oxidiert. 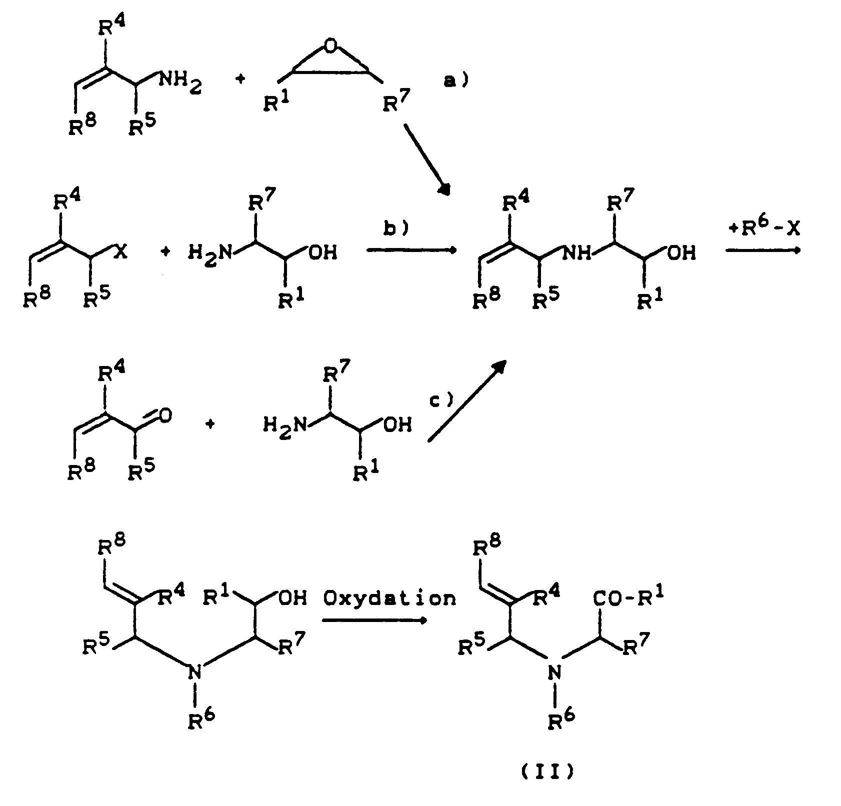
5. Enantiomerenreine Edukte der Formel (II) erhält man durch Alkylieren von N-acylierten Aminosäureestern mit Allylhalogeniden in Gegenwart starker Basen. Die Esterfunktion kann mit geeigneten Reduktionsmitteln
   a) zur Aldehydfunktion reduziert oder
   b) zum Alkohol reduziert und dann mit geeigneten Oxidationsmitteln zur Aldehydfunktion oxidiert werden.

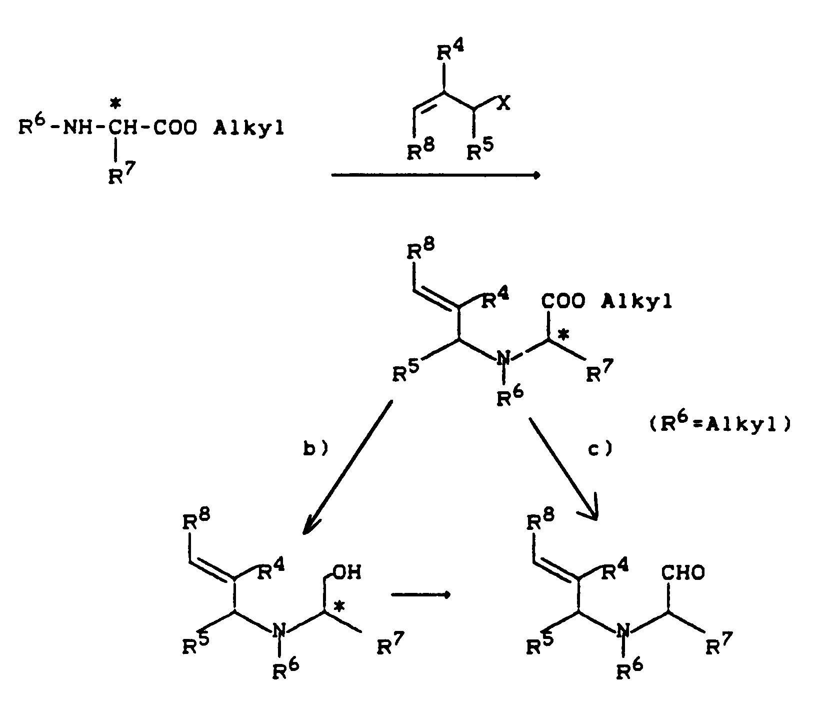

Als Beispiele für Edukte der Formel (II) seien genannt:
N-Allyl-N-(2-oxoethyl)-carbamidsäuremethylester,
N-Allyl-N-(2-oxoethyl)-carbamidsäureethylester,
N-Allyl-N-(2-oxoethyl)-carbamidsäureisopropylester,
N-Allyl-N-(2-oxoethyl)-carbamidsäure-tert.-butylester,
N-Allyl-N-(2-oxoethyl)-acetamid,
N-Allyl-N-(2-oxoethyl)-propionamid,
N-Allyl-N-(2-oxoethyl)-benzoesäureamid,
N-Allyl-N-(1-oxoprop-2-yl)-carbamidsäuremethylester,
N-Allyl-N-(1-oxoprop-2-yl)-carbamidsäureethylester,
N-Allyl-N-(1-oxoprop-2-yl)-carbamidsäurepropylester,
N-Allyl-N-(1-oxoprop-2-yl)-acetamid,
N-Allyl-N-(1-oxoprop-2-yl)-benzoesäureamid,
N-(Buten-3-yl)-N-(2-oxoethyl)-carbamidsäuremethylester,
N-(Buten-3-yl)-N-(2-oxoethyl)-carbamidsäureethylester,
N-(Buten-3-yl)-N-(2-oxoethyl)-carbamidsäureisopropylester,
N-(Buten-3-yl)-N-(2-oxoethyl)-carbamidsäure-tert.-butylester,
N-(Buten-3-yl)-N-(2-oxoethyl)-acetamid,
N-(Buten-3-yl)-N-(2-oxoethyl)-benzoesäureamid,
N-(2-Methylallyl)-N-(2-oxoethyl)-carbamidsäuremethylester,
N-(2-Methylallyl)-N-(2-oxoethyl)-carbamidsäureethylester,
N-(2-Methylallyl)-N-(2-oxoethyl)-acetamid,
N-(2-Methylallyl)-N-(2-oxoethyl)-benzoesäureamid,
N-(2-Fluorallyl)-N-(2-oxoethyl)-carbamidsäuremethylester,
N-(2-Fluorallyl)-N-(2-oxoethyl)-carbamidsäureethylester,
N-(2-Fluorallyl)-N-(2-oxoethyl)-acetamid,
N-(2-Fluorallyl)-N-(2-oxoethyl)-benzoesäureamid,
N-(2-Chlorallyl)-N-(2-oxoethyl)-carbamidsäuremethylester,
N-(2-Chlorallyl)-N-(2-oxoethyl)-carbamidsäureethylester,
N-(2-Chlorallyl)-N-(2-oxoethyl)-acetamid,
N-(2-Chlorallyl)-N-(2-oxoethyl)-benzoesäureamid,
N-(2-Bromallyl)-N-(2-oxoethyl)-carbamidsäuremethylester,
N-(2-Bromallyl)-N-(2-oxoethyl)-carbamidsäureethylester,
N-(2-Bromallyl)-N-(2-oxoethyl)-acetamid,
N-(2-Bromallyl)-N-(2-oxoethyl)-benzoesäureamid,
N-(2-Ethoxycarbonylallyl)-N-(2-oxoethyl)-carbamidsäuremethylester,
N-(2-Ethoxycarbonylallyl)-N-(2-oxoethyl)-carbamidsäureethylester,
N-(2-Ethoxycarbonylallyl)-N-(2-oxoethyl)-acetamid,
N-(2-Ethoxycarbonylallyl)-N-(2-oxoethyl)-benzoesäureamid,
N-(2-Oxoethyl)-N-(2-phenylallyl)-carbamidsäureethylester,
N-(2-Buten-1-yl)-N-(2-oxoethyl)-carbamidsäuremethylester,
N-(2-Buten-1-yl)-N-(2-oxoethyl)-carbamidsäureethylester,
N-(2-Buten-1-yl)-N-(2-oxoethyl)-acetamid,
N-(2-Buten-1-yl)-N-(2-oxoethyl)-benzoesäureamid,
N-(3-Chlorallyl)-N-(2-oxoethyl)-carbamidsäuremethylester,
N-(3-Chlorallyl)-N-(2-oxoethyl)-carbamidsäureethylester,
N-(3-Chlorallyl)-N-(2-oxoethyl)-acetamid,
N-(3-Chlorallyl)-N-(2-oxoethyl)-benzoesäureamid,
N-(3-Phenylallyl)-N-(2-oxoethyl)-carbamidsäuremethylester,
N-(3-Phenylallyl)-N-(2-oxoethyl)-carbamidsäurethylester,
N-(3-Phenylallyl)-N-(2-oxoethyl)-acetamid,
N-(3-Phenylallyl)-N-(2-oxoethyl)-benzoesäureamid,
N-(3-Ethoxycarbonylallyl)-N-(2-oxoethyl)-carbamidsäuremethylester,
N-(3-Ethoxycarbonylallyl)-N-(2-oxoethyl)-carbamidsäureethylester,
N-(3-Ethoxycarbonylallyl)-N-(2-oxoethyl)-acetamid,
N-Allyl-N-(2-oxopropyl)-carbamidsäureethylester,
N-Allyl-N-(2-oxopropyl)-acetamid,
N-Allyl-N-(2-oxopropyl)-benzoesäureamid,
N-Allyl-N-(2-oxo-2-phenylethyl)-acetamid,
N-Allyl-N-(2-ethoxycarbonyl-2-oxoethyl)-acetamid,
N-Allyl-N-benzyl-N-2-(oxopropyl)-amin,
N-Benzyl-N-(2-methylallyl)-N-(2-oxoethyl)-amin,
Die Ausgangsverbindungen der Formel (III) sind literaturbekannt und größtenteils kommerziell erhältlich.

Als Beispiele für Edukte der Formel (III) seien genannt:
Sarkosin, N-Ethylglycin, N-Propylglycin, N-Isopropylglycin, N-Phenylglycin, N-Benzylglycin, N-Methylalanin, N-Phenylalanin, N-Methylphenylglycin, N-Benzylphenylglycin, Azetidin-2-carbonsäure, Prolin, trans-4-Hydroxyprolin, Piperidin-2-carbonsäure, Thiazolidin-4-carbonsäure, 5,5-Dimethylthiazolidin-4-carbonsäure, Sarkosinmethylester, Sarkosinethylester, N-Benzylglcyinmethylester, N-Benzylglycinethylester.

Die Umsetzung von (II) mit (III) nach dem erfindungsgemäßen Verfahren wird in einem Lösungsmittel durchgeführt. Es können Kohlenwasserstoffe wie Benzol, Toluol, Xylole oder Tetralin, Ether wie Dioxan, Dibutylether, Dimethoxyethan, Diethylenglykoldimethylether, Diethylenglykoldiethylether, Alkohole wie Butanol, Pentanol, Ethylenglykol, Ethylenglykolmonomethylether und Ethylenglykolmonoethylether, und dipolar aprotische Lösungsmittel wie Dimethylformamid, Dimethylsulfoxid, N-Methylpyrrolidon und Sulfolan verwendet werden. Besonders bevorzugt sind Toluol, Xylole und Dimethylformamid.

Die Reaktionstemperatur kann in einem größeren Bereich variiert werden. Im allgemeinen führt man die Umsetzungen zwischen 20°C und 200°C, vorzugsweise zwischen 80°C und 150°C durch.

Die Umsetzung kann bei Normaldruck, aber auch bei erhöhtem Druck durchgeführt werden. Allgemein arbeitet man bei Drucken zwischen etwa 1 bar und 100 bar, vorzugsweise zwischen etwa 1 bar und 10 bar.

Bei der Durchführung des erfindungsgemäßen Verfahrens setzt man auf 1 Mol ungesättigter Carbonylverbindung (II) 0,5 bis 6 Mol, vorzugsweise 0,5 bis 2 Mol Aminosäurederivat (III) ein.

Die Reaktion kann so durchgeführt werden, daß die ungesättigte Carbonylverbindung zu einer Suspension oder Lösung des Aminosäurederivates (III) in einem der angegebenen Lösungsmittel zugetropft wird. Man kann aber auch beide Komponenten in einem Lösungsmittel vorlegen und die Reaktion im angegebenen Temperaturbereich durchführen. Das bei der Reaktion freiwerdende Reaktionswasser kann mit dem Lösungsmittel als Azeotrop abdestilliert werden. Der Reaktionsverlauf ist durch die stattfindende CO₂-Entwicklung gut zu verfolgen. Die Aufarbeitung erfolgt, gegebenenfalls nach Abtrennen nicht umgesetzter Aminosäure (III), durch Entfernen des Lösungsmittels und Destillation. Es ist auch möglich, die basischen Produkte mit einer Säure, wie zum Beispiel Salzsäure, aus dem organischen Lösungsmittel zu extrahieren, um neutrale Verunreinigungen abzutrennen.

In einem weiteren Schritt des erfindungsgemäßen Verfahrens können die Substituenten R² und R⁶, soweit sie Schutzgruppenfunktion besitzen, abgespalten werden.

Acylreste werden hydrolytisch entfernt. Zur Hydrolyse eignen sich starke Säuren oder starke Basen. Zur sauren Hydrolyse verwendet man bevorzugt wäßrige Salzsäure, Bromwasserstoffsäure oder Trifluoressigsäure. Die basische Hydrolyse führt man mit Alkalimetallhydroxiden oder Erdalkalimetallhydroxiden aus, bevorzugt sind Lithiumhydroxid, Natriumhydroxid, Kaliumhydroxid, Calciumhydroxid und Bariumhydroxid. Als Lösungsmittel dienen Wasser und Alkohole, bevorzugt sind Wasser, Ethanol oder Gemische aus diesen Lösungsmitteln. Die Hydrolyse kann bei Temperaturen zwischen 0° und 200°C, bevorzugt zwischen 20° und 140°C durchgeführt werden. Dabei arbeitet man bei Drucken zwischen etwa 1 bar und 100 bar, bevorzugt zwischen etwa 1 bar und 10 bar.

Sind die Reste R² oder R⁶ Benzylreste, so können diese Reste hydrogenolytisch abgespalten werden. Als Lösungsmittel können Wasser, Alkohole, Carbonsäuren, alkoholische Salzsäure, cyclische Ether oder deren Gemische verwendet werden. Als Katalysatoren verwendet man Palladium, sowohl als Schwamm als auch auf Trägern wie Aktivkohle, Calciumcarbonat oder Bariumsulfat, und Palladiumhydroxid auf Aktivkohle. Man arbeitet bei Temperaturen zwischen etwa 0° und 200° und Wasserstoffdrucken von 1 bar bis 200 bar.

Das erfindungsgemäße Verfahren beinhaltet darüber hinaus die Überführung der Reste R² und R⁶, sofern es sich um Acylreste handelt, in Alkylreste durch Reduktion. Die Reduktion kann sowohl katalytisch als auch mit Hydriden oder komplexen Hydriden der Elemente der dritten Hauptgruppe erfolgen. Bevorzugt erfolgt die Reduktion mit Diboran, Lithiumaluminiumhydrid und Natriumborhydrid, im letzten Fall unter Zusatz von Lewis-Säuren wie Titantetrachlorid, Aluminiumtrichlorid oder Bortrifluorid.

Die Reaktion erfolgt in inerten organischen Lösungsmitteln wie Ethern, zum Beispiel Diethylether, Dibutylether, Methyl-tert.-butylether, Tetrahydrofuran, Dioxan oder Ethylenglykoldimethylether oder Kohlenwasserstoffen wie Toluol oder Xylol. Die Temperaturen können zwischen etwa 0° und 200°C variiert werden. Zur Erzielung hoher Reaktionstemperaturen kann man bei Drucken bis zu 100 bar arbeiten.

Bevorzugt reduziert man mit Lithiumaluminiumhydrid oder Natriumborhydrid/Bortrifluorid-Etherat in Tetrahydrofuran oder Dioxan bei der Rückflußtemperatur des Lösungsmittels.

Die erfindungsgemäßen Verbindungen dienen als Ausgangssubstanzen für antibakteriell wirksame Chinolon- bzw. Naphthyridoncarbonsäuren.

So läßt sich beispielsweise 8-Chlor-1-cyclopropyl-6,7-difluor-1,4-dihydro-4-oxo-3-chinolincarbonsäure mit 2-Methyl-2,7-diazabicyclo[3.3.0]octan zur 8-Chlor-1-cyclopropyl-6-fluor-1,4-dihydro-7-(2-methyl-2,7-diazabicyclo[3.3.0]oct-7-yl)-4-oxo-3-chinolincarbonsäure umsetzen, die eine hohe antibakterielle Aktivität aufweist.

### Beispiel 1

### 2,7-Diazabicyclo[3.3.0]octan

a) N-(2,2-Dimethoxyethyl)-carbamidsäureethylester H₅C₂OOC-NH-CH₂-CH(OCH₃)₂
   Zu 214 g (2 Mol) Aminoacetaldehyddimethylacetal in 1 l Toluol und 90 g NaOH in 500 ml Wasser tropft man 214 g (2 Mol) Chlorameisensäureethylester bei 10°C. Man rührt noch 2 Stunden bei Raumtemperatur, trennt die wäßrige Phase ab, sättigt sie mit Kochsalz und extrahiert mit Toluol. Die Toluollösungen werden über Magnesiumsulfat getrocknet, eingeengt und destilliert.
   Ausbeute: 338 g (95,4 % der Theorie)
   Siedepunkt: 60°C/0,03 mbar
b) N-Allyl-N-(2,2-dimethoxyethyl)-carbamidsäureethylester 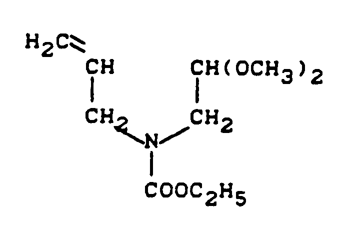 Man legt 500 g (2,82 Mol) N-(2,2-Dimethoxyethyl)carbamidsäureethylester, 625 g gepulvertes Kaliumhydroxid und 10 g Triethylbenzylammoniumchlorid in 2,7 l Toluol vor und tropft 345 g (2,85 Mol) Allylbromid bei Raumtemperatur hinzu. Man rührt über Nacht bei Raumtemperatur, saugt die Salze ab, wäscht das Filtrat einmal mit gesättigter Kochsalzlösung, trocknet über Kaliumcarbonat, engt ein und destilliert.
   Ausbeute: 582 g (95 % der Theorie)
   Siedepunkt: 64°C/0,1 mbar
c) N-Allyl-N-(2-oxoethyl)-carbamidsäureethylester 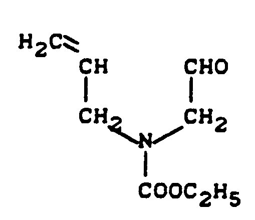 Man erhitzt 68 g (0,313 Mol) N-Allyl-N-(2,2-dimethoxyethyl)-carbamidsäureethylester mit 150 ml Ameisensäure eine Stunde auf 100°C. Man gießt auf Eis, extrahiert mehrfach mit Methylenchlorid, wäscht die organischen Phasen mit Natriumhydrogencarbonatlösung, trocknet über Magnesiumsulfat, engt ein und destilliert.
   Ausbeute: 46,7 g (87,2 % der Theorie)
   Siedepunkt: 58°C / 0,09 mbar
d) N-Benzylglycin 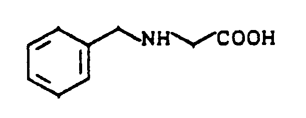 Man erhitzt 225,8 g (1,17 Mol) N-Benzylglycinethylester (J. Am. Chem. Soc. 72, 1238 (1950)) in 600 ml Wasser über Nacht unter Rückfluß. Man saugt auskristallisiertes Produkt ab und extrahiert das Filtrat einmal mit tert.-Butylmethylether. Man engt die wäßrige Phase ein und trocknet die erhaltenen Kristalle zusammen mit dem abfiltrierten Produkt im Exsikkator über Phosphorpentoxid.
   Ausbeute: 184 g (95 % der Theorie)
   Schmelzpunkt: 199°C
e) 2-Benzyl-2,7-diazabicyclo[3.3.0]octan-7-carbonsäurethylester 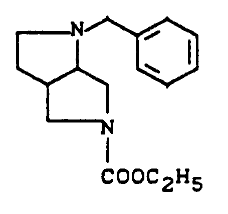 Man erhitzt 42,8 g (0,25 Mol) N-Allyl-N-(2-oxoethyl)-carbamidsäureethylester mit 41,3 g (0,25 Mol) N-Benzylglycin in 750 ml Toluol über Nacht unter Rückfluß. Man engt ein und destilliert den Rückstand.
   Ausbeute: 59,6 g (87 % der Theorie)
   Siedepunkt: 140°C / 0,09 mbar
f) 2,7-Diazabicyclo[3.3.0]octan-7-carbonsäureethylester 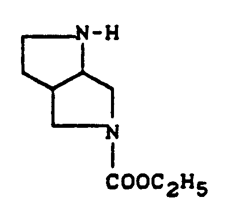 Man hydriert 21,2 g (77,3 mmol) 2-Benzyl-2,7-diazabicyclo[3.3.0]octan-7-carbonsäureethylester in 400 ml Ethanol an 3 g Palladium-Aktivkohle (10 % Pd) bei 100°C und 100 bar. Der Katalysator wird abfiltriert, das Filtrat eingeengt und der Rückstand destilliert.
   Ausbeute: 10,3 g (72,3 % der Theorie)
   Siedepunkt: 82 - 92°C / 0,1 mbar
g) 2,7-Diazabicyclo[3.3.0]octan 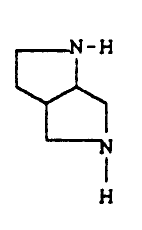 Man erhitzt 9 g (48,8 mmol) 2,7-Diazabicyclo[3.3.0]-octan-7-carbonsäureethylester mit 32 g (100 mmol) Ba(OH)₂ · 8H₂O in 140 ml Wasser über Nacht unter Rückfluß. Man sättigt den Ansatz mit Kaliumcarbonat, saugt Bariumcarbonat ab und extrahiert das Filtrat zehnmal mit je 100 ml Chloroform. Man trocknet über Kaliumcarbonat, engt ein und destilliert den Rückstand.
   Ausbeute: 3,4 g (62 % der Theorie)
   Siedepunkt: 70°C / 6 mbar

### Beispiel 2

### 2-Benzyl-2,7-diazabicyclo[3.3.0]octan

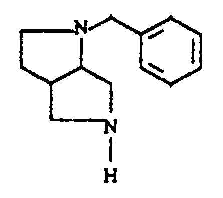

Man erhitzt 55,6 g (0,2 Mol) 2-Benzyl-2,7-diazabicyclo[3.3.0]octan-7-carbonsäureethylester mit 300 ml konzentrierter Salzsäure über Nacht unter Rückfluß. Dann stellt man den Ansatz mit Kaliumcarbonat alkalisch, extrahiert fünfmal mit je 100 ml Chloroform, trocknet über Kaliumcarbonat, engt ein und destilliert.
Ausbeute: 31 g (76,6 % der Theorie)
Siedepunkt: 105°C / 0,45 mbar

### Beispiel 3

### 2,7-Diazabicyclo[3.3.0]octan-2-carbonsäureethylester

a) 2-Benzyldiazabicyclo[3.3.0]octan-7-carbonsäuretert.-butylester 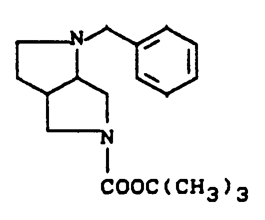 Man löst 20,2 g (0,1 Mol) 2-Benzyl-2,7-diazabicyclo[3.3.0]octan in 125 ml tert.-Butanol, setzt eine Lösung von 4,2 g Natriumhydroxid in 100 ml Wasser hinzu und tropft bei Raumtemperatur 23 g (0,105 Mol) Pyrokohlensäure-di-tert.-butylester hinzu. Man rührt über Nacht bei Raumtemperatur, extrahiert fünfmal mit je 100 ml Chloroform, trocknet über Kaliumcarbonat, engt ein und destilliert.
   Ausbeute: 24,8 g (82 % der Theorie)
   Siedepunkt: 145 - 149°C / 0,8 mbar
b) 2,7-Diazabicyclo[3.3.0]octan-7-carbonsäure-tert.-butylester 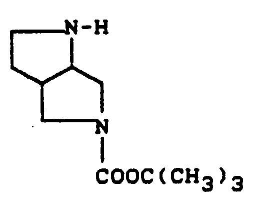 Man hydriert 24 g (79,4 mmol) 2-Benzyldiazabicyclo[3.3.0]octan-7-carbonsäure-tert.-butylester in 400 ml Ethanol an 3 g Palladium-Aktivkohle (10 % Pd) bei 100°C und 100 bar. Der Katalysator wird abfiltriert, das Filtrat eingeengt und der Rückstand destilliert.
   Ausbeute: 13,1 g (77,7 % der Theorie)
   Siedepunkt: 87°C / 0,1 mbar
c) 2,7-Diazabicyclo[3.3.0]octan-2,7-dicarbonsäure-2-ethylester-7-tert.-butylester 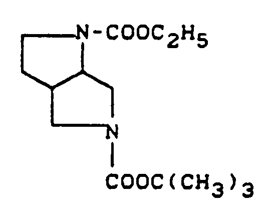 Man löst 13 g (61,2 mmol) 2,7-Diazabicyclo[3.3.0]octan-7-carbonsäure-tert.-butylester in 100 ml Toluol, setzt eine Lösung von 3 g Natriumhydroxid in 20 ml Wasser hinzu und tropft bei Raumtemperatur 7 g (64,5 mmol) Chlorameisensäureethylester hinzu. Man rührt drei Stunden bei Raumtemperatur, trennt die wäßrige Phase ab und extrahiert sie zweimal mit je 100 ml Methylenchlorid. Man trocknet die organischen Lösungen über Magnesiumsulfat, engt ein und destilliert.
   Ausbeute: 16 g (91,9 % der Theorie)
   Siedepunkt: 125°C / 0,13 mbar
d) 2,7-Diazabicyclo[3.3.0]octan-2-carbonsäureethylester 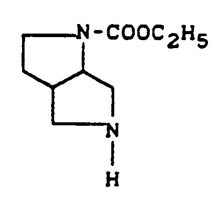 Man erhitzt 15,2 g (53,5 mmol) 2,7-Diazabicyclo[3.3.0]-octan-2,7-dicarbonsäure-2-ethylester-7-tert.-butylester in 100 ml Chloroform mit 10,5 g (55,3 Mol) para-Toluolsulfonsäure fünf Stunden unter Rückfluß. Man wäscht mit 50 ml 10%iger Natronlauge, trocknet die organische Phase über Kaliumcarbonat, engt ein und destilliert.
   Ausbeute: 9,5 g (96,4 % der Theorie)
   Siedepunkt: 80 - 90°C / 0,1 mbar

### Beispiel 4

### 2-Methyl-2,7-diazabicyclo[3.3.0]octan

a) 2-Methyl-2,7-diazabicyclo[3.3.0]octan-7-carbonsäuremethylester 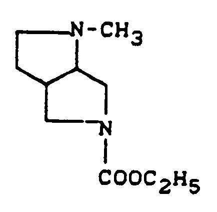 Man erhitzt 8,6 g (50 mmol) N-Allyl-N-(2-oxoethyl)carbamidsäureethylester mit 4,5 g (50 mmol) Sarkosin in 200 ml Toluol über Nacht unter Rückfluß. Man engt ein und destilliert den Rückstand.
   Ausbeute: 7,5 g (75,7 % der Theorie)
   Siedepunkt: 80 - 82°C / 0,1 mbar
b) 2-Methyl-2,7-diazabicyclo[3.3.0]octan 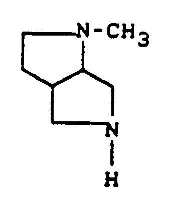 Man erhitzt 9 g (45,4 mmol) 2-Methyl-2,7-diazabicyclo[3.3.0]octan-7-carbonsäureethylester mit 50 ml konzentrierter Salzsäure über Nacht unter Rückfluß. Man stellt den Ansatz mit Kaliumcarbonat alkalisch, extrahiert zehnmal mit je 50 ml Chloroform, trocknet über Kaliumcarbonat, engt ein und destilliert.
   Ausbeute: 4,5 g (78 % der Theorie)
   Siedepunkt: 72°C / 25 mbar

### Beispiel 5

### 2-Phenyl-2,7-diazabicyclo[3.3.0]octan

a) 2-Phenyl-2,7-diazabicyclo[3.3.0]octan-7-carbonsäureethylester 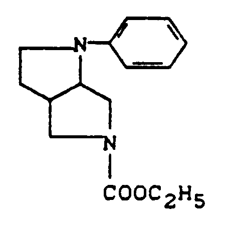 Man erhitzt 8,6 g (50 mmol) N-Allyl-N-(2-oxoethyl)carbamidsäureethylester und 7,6g(50 mmol) Phenylglycin in 200 ml Toluol über Nacht unter Rückfluß. Man dekantiert von harzigem Material, engt ein und destilliert den Rückstand.
   Ausbeute: 8,1 g (62,2 % der Theorie)
   Siedepunkt: 151°C / 0,12 mbar
b) 2-Phenyl-2,7-diazabicyclo[3.3.0]octan 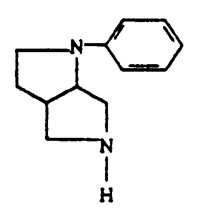 Man erhitzt 7,6 g (31,6 mmol) 2-Phenyl-2,7-diazabicyclo[3.3.0]octan-7-carbonsäureethylester mit 50 ml konzentrierter Salzsäure über Nacht unter Rückfluß. Man engt ein, nimmt den Rückstand in 50 ml 10%iger Natronlauge auf und extrahiert fünfmal mit je 50 ml Chloroform. Man trocknet über Kaliumcarbonat, engt ein und destilliert den Rückstand.
   Ausbeute: 3,7 g (62 % der Theorie)
   Siedepunkt: 103°C / 0,08 mbar

### Beispiel 6

### 3-Methyl-2,7-diazabicyclo[3.3.0]octan

a) N-Benzylalanin 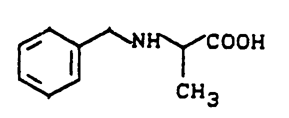 Man erhitzt 333 g (1,72 mol) N-Benzylalaninmethylester (J .Chem. Soc. 4374 (1952)) mit 860 ml Wasser über Nacht unter Rückfluß. Man saugt ausgeschiedenes Produkt ab und extrahiert das Filtrat einmal mit tert.-Butylmethylether. Die wäßrige Lösung wird eingeengt und die erhaltenen Kristalle mit der ersten Kristallfraktion im Exsikkator über Phosphorpentoxid getrocknet.
   Ausbeute: 280 g (91 % der Theorie)
   Schmelzpunkt: 270 - 276°C (Zersetzung)
b) 2-Benzyl-3-methyl-2,7-diazabicyclo[3.3.0]octan-7-carbonsäureethylester 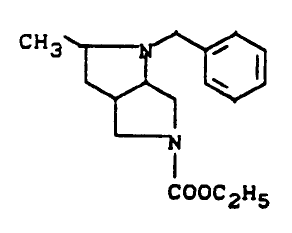 Man erhitzt 42,8 g (0,25 mol) N-Allyl-N-(2-oxoethyl)-carbamidsäureethylester mit 44,8 g (0,25 mol) N-Benzylalanin in 750 ml Toluol über Nacht unter Rückfluß. Man engt ein und destilliert den Rückstand zweimal.
   Ausbeute: 32 g (44,4 % der Theorie)
   Siedepunkt: 128-133°C/0,06 mbar
   Das Produkt besteht zu 96 % aus einem Stereoisomeren.
c) 3-Methyl-2,7-diazabicyclo[3.3.0]octan-7-carbonsäureethylester 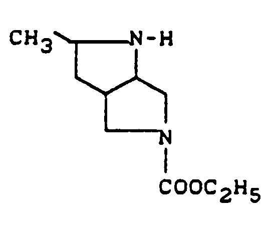 Man hydriert 32 g (0,11 mol) 2-Benzyl-3-methyl-2,7-diazabicyclo[3.3.0]octan-7-carbonsäureethylester in 560 ml Ethanol an 4,5 g Palladium-Aktivkohle bei 100°C und 100 bar. Der Katalysator wird abgesaugt, das Filtrat eingeengt und der Rückstand destilliert.
   Ausbeute: 17,1 g (77,7 % der Theorie)
   Siedepunkt: 140-145°C/8 mbar
d) 3-Methyl-2,7-diazabicyclo[3.3.0]octan 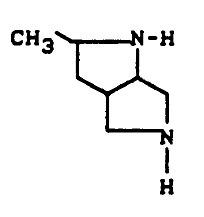 Man erhitzt 17 g (85,7 mmol) 3-Methyl-2,7-diazabicyclo[3.3.0]octan-7-carbonsäureethylester mit 100 ml konzentrierter Salzsäure über Nacht unter Rückfluß. Man engt ein, nimmt in 50 ml Wasser auf, stellt mit Kaliumcarbonat alkalisch und extrahiert zehnmal mit je 50 ml Chloroform. Man trocknet über Kaliumcarbonat, engt ein und destilliert den Rückstand.
   Ausbeute: 6 g (55 % der Theorie)
   Siedepunkt: 68-70°C/6 mbar

### Beispiel 7

### 2,3-Dimethyl-2,7-diazabicyclo[3.3.0]octan

a) 2,3-Dimethyl-2,7-diazabicyclo[3.3.0]octan-7-carbonsäureethylester 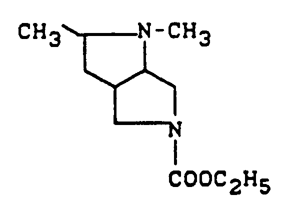 Man erhitzt 17,2 g (0,1 mol) N-Allyl-N-(2-oxoethyl)-carbamidsäureethylester mit 10,5 g (0,1 mol) N-Methylalanin in 300 ml Toluol über Nacht unter Rückfluß. Man engt ein und destilliert den Rückstand.
   Ausbeute: 11,3 g (53,2 % der Theorie)
   Siedepunkt: 81°C/0,25 mbar
b) 2,3-Dimethyl-2,7-diazabicyclo[3.3.0]octan 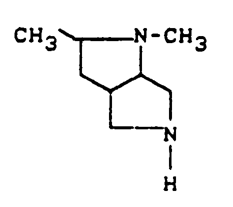 Man erhitzt 7,25 g (34,2 mmol) 2,3-Dimethyl-2,7-diazabicyclo[3.3.0]octan-7-carbonsäureethylester mit 50 ml konzentrierter Salzsäure über Nacht unter Rückfluß. Man stellt mit Kaliumcarbonat alkalisch, extrahiert zehnmal mit je 50 ml Chloroform, trocknet über Kaliumcarbonat, engt ein und destilliert den Rückstand.
   Ausbeute: 3 g (62,5 % der Theorie)
   Siedepunkt: 72-74°C/10 mbar

### Beispiel 8

### 2,8-Dimethyl-2,7-diazabicyclo[3.3.0]octan

a) N-(1,1-Dimethoxyprop-2-yl)-carbamidsäureethylester 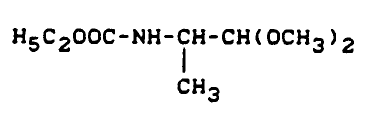 Zu 86,2 g (0,72 mol) 2-Aminopropionaldehyddimethylacetal in 350 ml Toluol und 32 g (0,8 mol) NaOH in 300 ml Wasser tropft man unter Eiskühlung 80 g (0,73 mol) Chlorameisensäureethylester. Man rührt noch 2 Stunden bei Raumtemperatur, trennt die organische Phase ab, extrahiert die wäßrige Phase mit Toluol und trocknet die Toluollösungen über K₂CO₃. Man engt ein und destilliert.
   Ausbeute: 132 g (95 % der Theorie)
   Siedepunkt: 55°C/0,06 mbar
b) N-Allyl-N-(1,1-dimethoxyprop-2-yl)-carbamidsäureethylester 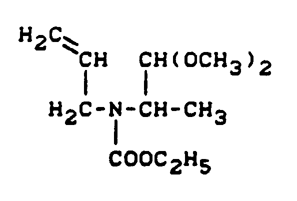 Man legt 151 g (0,79 mol) N-(1,1-Dimethoxyprop-2-yl)-carbamidsäureethylester, 175 g gepulvertes Kaliumhydroxid und 2,8 g Triethylbenzylammoniumchlorid in 750 ml Toluol vor und tropft bei Raumtemperatur 94 g (0,777 mol) Allylbromid hinzu. Nach Rühren über Nacht bei Raumtemperatur wurden weitere 10 g (82,6 mmol) Allylbromid zugetropft und ein Tag bei Raumtemperatur gerührt. Man setzt Wasser zu bis alle Salze in Lösung gegangen sind, trennt die wäßrige Phase ab und extrahiert sie zweimal mit je 150 ml Toluol. Man trocknet über K₂CO₃, engt ein und destilliert den Rückstand.
   Ausbeute: 173 g (94,7 % der Theorie)
   Siedepunkt: 68°C/0,1 mbar
c) Allyl-(1,1-dimethoxyprop-2-yl)-amin 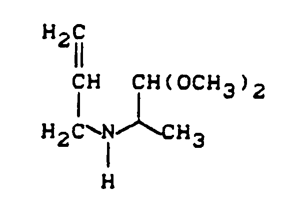 Zu 12 g (0,1 mol) 1,1-Dimethoxyaceton in 100 ml Ethanol setzt man 20 g Molsieb zu und tropft dann 7 g (0,12 mol) Allylamin hinzu. Man läßt über Nacht bei Raumtemperatur stehen, dekantiert vom Molsieb, kühlt im Eisbad auf 0°C und versetzt mit 4 g (0,1 mol) Natriumborhydrid in kleinen Portionen. Man rührt über Nacht bei Raumtemperatur, engt ein, nimmt in 100 ml Wasser auf, sättigt mit Kaliumcarbonat und extrahiert fünfmal mit je 100 ml Chloroform. Man trocknet über Kaliumcarbonat, engt ein und destilliert.
   Ausbeute: 10,3 g (64,7 % der Theorie)
   Siedepunkt: 75°C/25 mbar
d) N-Allyl-N-(1,1-dimethoxyprop-2-yl)-carbamidsäureethylester 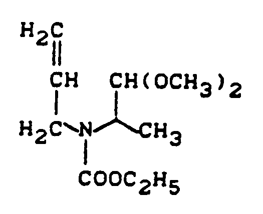 Man legt 125 g (0,785 mol) Allyl-(1,1-dimethoxyprop-2-yl)amin in 400 ml Toluol vor, setzt eine Lösung von 40 g Natriumhydroxid in 200 ml Wasser hinzu, kühlt im Eisbad auf 0°C und tropft 95 g (0,876 mol) Chlorameisensäureethylester hinzu. Anschließend rührt man drei Stunden bei Raumtemperatur, trennt die wäßrige Phase ab und extrahiert sie zweimal mit je 100 ml Toluol. Man trocknet über Kaliumcarbonat, engt ein und destilliert.
   Ausbeute: 170,8 g (94 % der Theorie)
   Siedepunkt: 55°C/0,05 mbar
e) N-Allyl-N-(1-oxoprop-2-yl)-carbamidsäureethylester 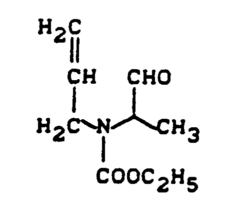 Man erhitzt 182 g (0,787 mol) N-Allyl-N-(1,1-dimethoxyprop-2-yl)-carbamidsäureethylester in 1,5 l Wasser mit 80 ml Ameisensäure drei Stunden unter Rückfluß. Man sättigt den Ansatz mit Kochsalz, trennt die organische Phase ab und extrahiert die wäßrige Phase zweimal mit je 500 ml Methylenchlorid. Die organischen Lösungen werden mit gesättigter Natriumhydrogencarbonatlösung neutral gewaschen, über Magnesiumsulfat getrocknet, eingeengt und destilliert.
   Ausbeute: 134 g (91,9 % der Theorie)
   Siedepunkt: 65°C/0,23 mbar
f) 2,8-Dimethyl-2,7-diazabicyclo[3.3.0]octan-7-carbonsäureethylester 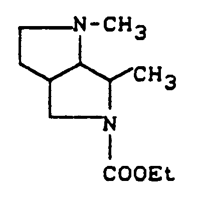 Man erhitzt 18,5 g (0,1 mol) N-Allyl-N-(1-oxoprop-2-yl)-carbamidsäureethylester mit 9 g (0,1 mol) Sarkosin in 300 ml Toluol über Nacht am Wasserabscheider unter Rückfluß. Man engt ein und destilliert den Rückstand.
   Ausbeute: 17 g (80 % der Theorie)
   Siedepunkt: 140-150° C/8 mbar
g) 2,8-Dimethyl-2,7-diazabicyclo[3.3.0]octan 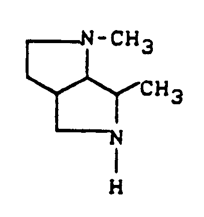 Man erhitzt 16,9 g (79,6 mol) 2,8-Dimethyl-2,7-diazabicyclo[3.3.0]octan-7-carbonsäureethylester mit 130 ml konzentrierter Salzsäure über Nacht unter Rückfluß. Man engt ein, nimmt in 50 ml Wasser auf, stellt mit Kaliumcarbonat alkalisch und extrahiert fünfmal mit je 50 ml Chloroform. Man trocknet über Kaliumcarbonat, engt ein und destilliert den Rückstand.
   Ausbeute: 6,6 g (58,5 % der Theorie)
   Siedepunkt: 60-62°C/6 mbar

### Beispiel 9

### 5-Chlor-2-methyl-2,7-diazabicyclo[3.3.0]octan

a) N-(2-Chlorallyl)-N-(2,2-dimethoxyethyl)-carbamidsäureethylester 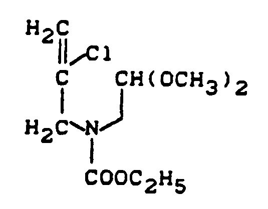 Man legt 115 g (0,65 mol) N-(2,2-Dimethoxyethyl)carbamidsäureethylester, 130 g gepulvertes Kaliumhydroxid und 2 g Triethylbenzylammoniumchlorid in 650 ml Toluol vor und tropft bei Raumtemperatur 142 g (0,7 mol) 2-Chlorallyliodid hinzu. Nach Rühren über Nacht zeigte ein Gaschromatogramm unvollständigen Umsatz, daher wurden erneut 65 g gepulvertes Kaliumhydroxid und 1 g Triethylbenzylammoniumchlorid zugesetzt und weitere 71 g (0,35 mol) 2-Chlorallyliodid zugetropft. Nach Rühren über Nacht bei Raumtemperatur wurden die Salze abgesaugt, das Filtrat mit gesättigter Kochsalzlösung gewaschen, über Magnesiumsulfat getrocknet, eingeengt und der Rückstand destilliert.
   Ausbeute: 140,9 g (86 % der Theorie)
   Siedepunkt: 92-97°C/0,8 mbar
b) N-(2-Chlorallyl)-N-(2-oxoethyl)-carbamidsäureethylester 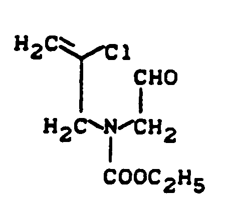 Man erhitzt 151 g (0,6 mol) N-(2-Chlorallyl)-N-(2,2-dimethoxyethyl)-carbamidsäureethylester mit 60 ml Ameisensäure in 1,2 l Wasser drei Stunden unter Rückfluß. Man sättigt mit Kochsalz, trennt die wäßrige Phase ab und extrahiert sie zweimal mit je 300 ml Methylenchlorid. Die organischen Phasen werden mit gesättigter Natriumhydrogencarbonatlösung neutral gewaschen, über Magnesiumsulfat getrocknet, eingeengt und destilliert.
   Ausbeute: 97,1 g (78 % der Theorie)
   Siedepunkt: 88-91°C/0,06 mbar
c) 5-Chlor-2-methyl-2,7-diazabicyclo[3.3.0]octan-7-carbonsäureethylester 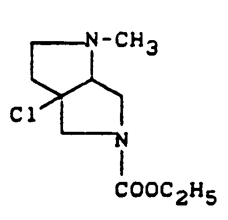 Man erhitzt 10,3 g (50 mmol) N-(2-Chlorallyl)-N-(2-oxoethyl)-carbamidsäureethylester mit 4,5 g (50 mmol) Sarkosin in 200 ml Toluol über Nacht unter Rückfluß. Man engt ein und destilliert den Rückstand.
   Ausbeute: 10,6 g (91 % der Theorie)
   Siedepunkt: 80°C/0,1 mbar
d) 5-Chlor-2-methyl-2,7-diazabicyclo[3.3.0]octan 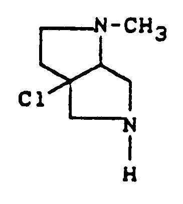 Man erhitzt 9,3 g (40 mmol) 5-Chlor-2-methyl-2,7-diazabicyclo[3.3.0]octan-7-carbonsäureethylester mit 50 ml konzentrierter Salzsaure über Nacht unter Rückfluß. Man engt ein, nimmt in 30 ml Wasser auf, stellt mit Kaliumcarbonat alkalisch und extrahiert fünfmal mit je 50 ml Chloroform. Man trocknet über Kaliumcarbonat, engt ein und destilliert.
   Ausbeute: 4,7 g (72 % der Theorie)
   Siedepunkt: 73°C/4 mbar

### Beispiel 10

### 5-Chlor-2,3-dimethyl-2,7-diazabicyclo[3.3.0]octan

a) 5-Chlor-2,3-dimethyl-2,7-diazabicyclo[3.3.0]octan-7-carbonsäureethylester 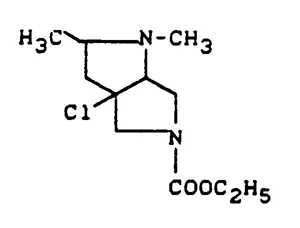 Man erhitzt 10,3 g (50 mmol) N-(2-Chlorallyl)-N-(2-oxoethyl)-carbamidsäureethylester mit 5,2 g (50,5 mmol) N-Methylalanin in 200 ml Toluol über Nacht unter Rückfluß. Man engt ein und destilliert den Rückstand.
   Ausbeute: 8,1 g (65,7 % der Theorie)
   Siedepunkt: 87°C/0,08 mbar
b) 5-Chlor-2,3-dimethyl-2,7-diazabicyclo[3.3.0]octan 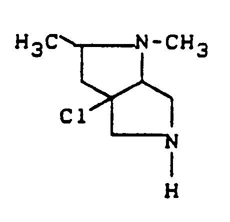 Man erhitzt 7,6 g (30,8 mmol) 5-Chlor-2,3-dimethyl-2,7-diazabicyclo[3.3.0]octan-7-carbonsäureethylester mit 30 ml konzentrierter Salzsäure über Nacht unter Rückfluß. Man engt ein, nimmt in 30 ml Wasser auf, stellt mit Kaliumcarbonat alkalisch, extrahiert fünfmal mit je 50 ml Chloroform, trocknet über Kaliumcarbonat, engt ein und destilliert.
   Ausbeute: 3,7 g (68,4 % der Theorie)
   Siedepunkt: 95-97°C/6 mbar

### Beispiel 11

### 1,4-Diazatricyclo[6.2.0.0^{2,6}]decan

a) 1,4-Diazatricyclo[6.2.0.0^{2,6}]decan-4-carbonsäureethylester 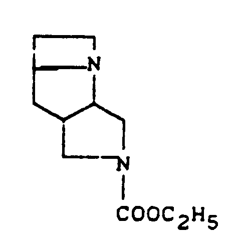 Man erhitzt 17,1 g (0,1 mol) N-Allyl-N-(2-oxoethyl)-carbamidsäureethylester mit 10 g (0,1 mol) Azetidin-2-carbonsäure in 200 ml Toluol über Nacht am Wasserabscheider unter Rückfluß. Man saugt nicht umgesetzte Aminosäure ab, engt das Filtrat ein und destilliert den Rückstand.
   Ausbeute: 13,8 g (65,6 % der Theorie)
   Siedepunkt: 108°C/0,35 mbar
b) 1,4-Diazatricyclo[6.2.0.0^{2,6}]decan 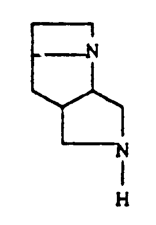 Man erhitzt 13,7 g (65,1 mmol) 1,4-Diazatricyclo[6.2.0.0^{2,6}]decan-4-carbonsäureethylester mit 42 g Ba(OH)₂.8H₂O in 150 ml Wasser über Nacht unter Rückfluß. Man versetzt mit Kaliumcarbonat, saugt Bariumcarbonat ab und extrahiert das Filtrat zehnmal mit je 100 ml Chloroform. Man trocknet über Kaliumcarbonat, engt ein und destilliert den Rückstand.
   Ausbeute: 5,3 g (58,9 % der Theorie)
   Siedepunkt: 85°C/6 mbar

### Beispiel 12

### 1,4-Diazatricyclo[6.3.0.0^{2,6}]undecan

a) 1,4-Diazatricyclo[6.3.0.0^{2,6}]undecan-4-carbonsäureethylester 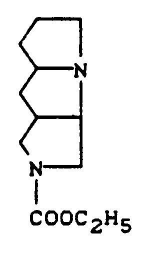 Man erhitzt 8,6 g (50 mmol) N-Allyl-N-(2-oxoethyl)carbamidsäureethylester mit 5,8 g (50 mmol) Prolin in 200 ml Toluol über Nacht unter Rückfluß. Man engt ein und destilliert den Rückstand.
   Ausbeute: 9,6 g (86 % der Theorie)
   Siedepunkt: 102-112°C/0,13-0,15 mbar
b) 1,4-Diazatricyclo[6.3.0.0^{2,6}]undecan 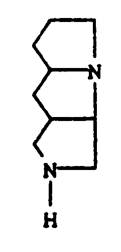 Man erhitzt 9 g (40 mmol) 1,4-Diazatricyclo[6.3.0.0^{2,6}]undecan-4-carbonsäureethylester mit 50 ml konzentrierter Salzsäure über Nacht unter Rückfluß. Man stellt mit Kaliumcarbonat alkalisch, extrahiert zehnmal mit je 50 ml Chloroform, trocknet über Kaliumcarbonat, engt ein und destilliert den Rückstand.
   Ausbeute: 4,9 g (80,5 % der Theorie)
   Siedepunkt: 50°C/0,05 mbar

### Beispiel 13

### 10-Hydroxy-1,4-diazatricyclo[6.3.0.0^{2,6}]undecan

a) 10-Hydroxy-1,4-diazatricyclo[6.3.0.0^{2,6}]undecan-4-carbonsäureethylester 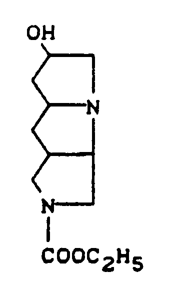 Man erhitzt 8,6 g (50 mmol) N-Allyl-N-(2-oxoethyl)carbamidsäureethylester mit 6,6 g (50 mmol) trans-4-Hydroxyprolin in 200 ml Dimethylformamid über Nacht auf 120°C. Man engt ein und destilliert den Rückstand.
   Ausbeute: 9,7 g (81 % der Theorie)
   Siedepunkt: 170°C/0,3 mbar
   Das Produkt besteht überwiegend aus zwei Stereoisomeren im Verhältnis 1:1.
b) 10-Hydroxy-1,4-diazatricyclo[6.3.0.0^{2,6}]undecan 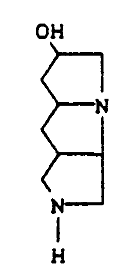 Man erhitzt 8 g (33,3 mmol) 10-Hydroxy-1,4-diazatricyclo[6.3.0.0^{2,6}]undecan-4-carbonsäureethylester mit 21 g Ba(OH)₂.8H₂O in 150 ml Wasser über Nacht unter Rückfluß. Man sättigt mit Kaliumcarbonat, saugt Bariumcarbonat ab und extrahiert zehnmal mit je 100 ml Chloroform. Man trocknet über Kaliumcarbonat, engt ein und destilliert.
   Ausbeute: 4,6 g (82 % der Theorie)
   Siedepunkt: 110-115°C/0,1 mbar

### Beispiel 14

### 1,4-Diazatricyclo[6.4.0.0^{2,6}]dodecan

a) 1,4-Diazatricyclo[6.4.0.0^{2,6}]dodecan-4-carbonsäureethylester 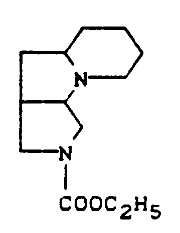 Man erhitzt 17,1 g (0,1 mol) N-Allyl-N-(2-oxoethyl)-carbamidsäureethylester mit 13 g (0,1 mol) Piperidin-2-carbonsäure in 200 ml Toluol über Nacht unter Rückfluß. Man engt ein und destilliert den Rückstand.
   Ausbeute: 20,8 g (87,2 % der Theorie)
   Siedepunkt: 105-112°/0,12 mbar
b) 1,4-Diazatricyclo[6.4.0.0^{2,6}]decan 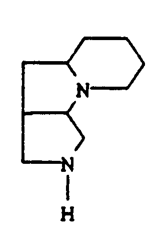 Man erhitzt 20,7 g (86,8 mmol) 1,4-Diazatricyclo[6.4.0.0^{2,6}]dodecan-4-carbonsäureethylester mit 250 ml konzentrierter Salzsäure über Nacht unter Rückfluß. Man engt ein, nimmt in 50 ml Wasser auf und stellt mit Kaliumcarbonat alkalisch. Man extrahiert zehnmal mit je 50 ml Chloroform, trocknet über Kaliumcarbonat, engt ein und destilliert den Rückstand.
   Ausbeute: 8,5 g (58,9 % der Theorie)
   Siedepunkt: 108°C/8 mbar

### Beispiel 15

### 10-Thia-1,4-diazatricyclo[6.3.0.0^{2,6}]undecan

a) 10-Thia-1,4-diazatricyclo[6.3.0.0^{2,6}]undecan-4-carbonsäureethylester 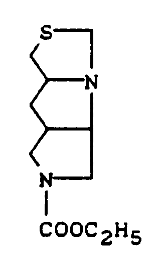 Man erhitzt 17,2 g (0,1 mol) N-Allyl-N-(2-oxoethyl)-carbamidsäureethylester mit 13,5 g (0,1 mol) Thiazolidin-4-carbonsäure in 300 ml Toluol über Nacht unter Rückfluß. Man engt ein und destilliert.
   Ausbeute: 20 g (82,5 % der Theorie)
   Siedepunkt: 155-156°C/0,5 mbar
b) 10-Thia-1,4-diazatricyclo[6.3.0.0^{2,6}]undecan 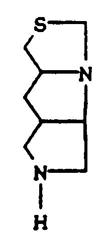 Man erhitzt 12,5 g (50 mmol) 10-Thia-1,4-diazatricyclo[6.3.0.0^{2,6}]undecan-4-carbonsäureethylester mit 32 g Ba(OH)₂.8H₂O in 225 ml Wasser über Nacht unter Rückfluß. Man versetzt mit Kaliumcarbonat, saugt Bariumcarbonat ab und extrahiert das Filtrat zehnmal mit je 100 ml Chloroform. Man trocknet über Kaliumcarbonat, engt ein und destilliert.
   Ausbeute: 6,2 g (72,8 % der Theorie)
   Siedepunkt: 90-94°C/0,05 mbar

### Beispiel 16

### 9,9-Dimethyl-10-thia-1,4-diazatricyclo[6.3.0.0^{2,6}]undecan

a) 9,9-Dimethyl-10-thia-1,4-diazatricyclo[6.3.0.0^{2,6}]undecan-4-carbonsäureethylester 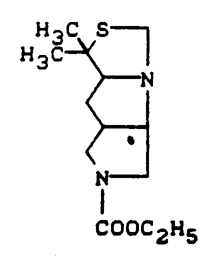 Man erhitzt 8,6 g (50 mmol) N-Allyl-N-(2-oxoethyl)carbamidsäureethylester mit 8,1 g (50 mmol) 5,5-Dimethylthiazolidin-4-carbonsäure in 200 ml Toluol über Nacht unter Rückfluß. Man engt ein und destilliert den Rückstand.
   Ausbeute: 8,4 g (62,2 % der Theorie)
   Siedepunkt: 141-155°C/0,03-0,05 mbar
b) 9,9-Dimethyl-10-thia-1,4-diazatricyclo[6.3.0.0^{2,6}]undecan 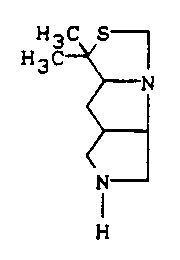 Man erhitzt 6 g (22,2 mmol) 9,9-Dimethyl-10-thia-1,4-diazatricyclo[6.3.0.0^{2,6}]undecan-4-carbonsäureethylester mit 12 g Ba(OH)₂.8H₂O in 100 ml Wasser über Nacht unter Rückfluß. Man versetzt mit Kaliumcarbonat, saugt Bariumcarbonat ab und extrahiert das Filtrat zehnmal mit je 100 ml Chloroform. Man trocknet über Kaliumcarbonat, engt ein und destilliert den Rückstand.
   Ausbeute: 2,25 g (51 % der Theorie)
   Siedepunkt: 83°C/0,02 mbar

### Beispiel 17

### 7-Methyl-2,7-diazabicyclo[3.3.0]octan

a) 2-Benzyl-7-methyl-2,7-diazabicyclo[3.3.0]octan 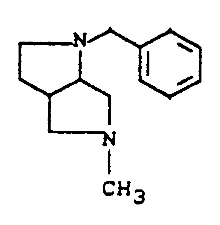 Zu 3,8 g (0,1 mol) Lithiumaluminiumhydrid in 100 ml absolutem Tetrahydrofuran tropft man 13,7 g (50 mmol) 2-Benzyldiazabicyclo[3.3.0]octan-7-carbonsäureethylester in 20 ml absolutem Tetrahydrofuran. Man erhitzt über Nacht unter Rückfluß und zersetzt nacheinander mit je 4 ml Wasser, 15 %iger Kalilauge und Wasser. Die anorganischen Salze werden abgesaugt und dreimal mit je 50 ml Tetrahydrofuran ausgekocht. Die organischen Lösungen werden eingeengt und der Rückstand destilliert.
   Ausbeute: 10,4 g (96 % der Theorie)
   Siedepunkt: 90-100°C/0,1 mbar
b) 7-Methyl-2,7-diazabicyclo[3.3.0]octan 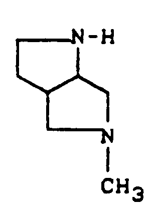 Man hydriert 10,3 g (47,6 mmol) 2-Benzyl-7-methyl-2,7-diazabicyclo[3.3.0]octan in 200 ml Ethanol an 2,5 g Palladium-Aktivkohle (10 % Pd) bei 100°C und 100 bar. Der Katalysator wird abgesaugt, das Filtrat eingeengt und der Rückstand destilliert.
   Ausbeute: 4,2 g (69,9 % der Theorie)
   Siedepunkt: 50-53°C/6 mbar

### Beispiel 18

### 2-Benzyl-8-methyl-2,7-diazabicyclo[3.3.0]octan

a) 2-Benzyl-8-methyl-2,7-diazabicyclo[3.3.0]octan-7-carbonsäureethylester 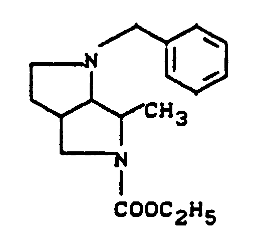 Man erhitzt 37 g (0,2 mol) N-Allyl-N-(1-oxo-2-propyl)-carbamidsäureethylester mit 33 g (0,2 mol) N-Benzylglycin in 500 ml Toluol am Wasserabscheider über Nacht unter Rückfluß. Man engt den Ansatz ein und destilliert den Rückstand.
   Ausbeute: 48,5 g (84 % der Theorie)
   Siedepunkt: 140-145°C/0,2 mbar
   Das Produkt ist gaschromatographisch ein einheitliches Stereoisomeres.
b) 2-Benzyl-8-methyl-2,7-diazabicyclo[3.3.0]octan 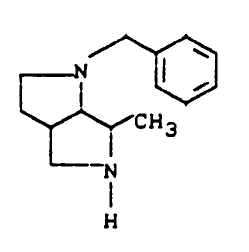 Man erhitzt 16 g (55 mmol) 2-Benzyl-8-methyl-2,7-diazabicyclo[3.3.0]octan-7-carbonsäureethylester mit 50 ml konzentrierter Salzsäure über Nacht unter Rückfluß. Man engt den Ansatz ein, löst in 50 ml Wasser und stellt mit Kaliumcarbonat alkalisch. Man extrahiert fünfmal mit je 50 ml Chloroform, trocknet über K₂CO₃, engt ein und destilliert den Rückstand.
   Ausbeute: 7,9 g (66,4 % der Theorie)
   Siedepunkt: 108-113°C/0,17 mbar

### Beispiel 19

### 8-Methyl-2,7-diazabicyclo[3.3.0]octan

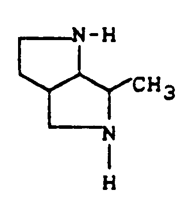

Man hydriert 7,8 g (36 mmol) 2-Benzyl-8-methyl-2,7-diazabicyclo[3.3.0]octan in 200 ml Ethanol an 2 g Palladium-Aktivkohle (10 % Pd) bei 100°C und 100 bar. Man saugt den Katalysator ab, engt das Filtrat ein und destilliert den Rückstand. Das Destillat kristallisiert.
Ausbeute: 3,3 g (72,7 % der Theorie)
Siedepunkt: 110°C/30 mbar
Schmelzpunkt: 72-75°C

### Beispiel 20

### 8-Methyl-2,7-diazabicyclo[3.3.0]octan-7-carbonsäureethylester

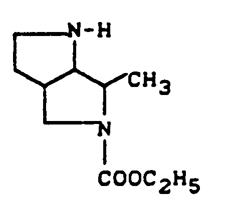

Man hydriert 16 g (55 mmol) 2-Benzyl-8-methyl-2,7-diazabicyclo[3.3.0]octan-7-carbonsäureethylester in 300 ml Ethanol an 3 g Palladium-Aktivkohle (10 % Pd) bei 100°C und 100 bar. Man saugt den Katalysator ab, engt das Filtrat ein und destilliert den Rückstand.
Ausbeute: 9,7 g (89 % der Theorie)
Siedepunkt: 100°C/0,1 mbar

### Beispiel 21

### 7,8-Dimethyl-2,7-diazabicyclo[3.3.0]octan

a) 2-Benzyl-7,8-dimethyl-2,7-diazabicyclo[3.3.0]octan 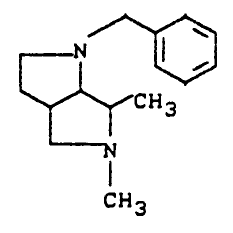 Zu 3,8 g (0,1 mol) Lithiumaluminiumhydrid in 100 ml absolutem Tetrahydrofuran tropft man 14,4 g (50 mmol) 2-Benzyl-8-methyl-2,7-diazabicyclo[3.3.0]octan-7-carbonsäureethylester in 20 ml absolutem Tetrahydrofuran und erhitzt anschließend über Nacht unter Rückfluß. Man zersetzt nacheinander mit je 4 ml Wasser, 15 %iger Kalilauge und Wasser, saugt die anorganischen Salze ab und kocht sie dreimal mit je 50 ml Tetrahydrofuran aus. Die organischen Lösungen werden eingeengt und der Rückstand destilliert.
   Ausbeute: 10,9 g (94,6 % der Theorie)
   Siedepunkt: 105°C/0,08 mbar
b) 7,8-Dimethyl-2,7-diazabicyclo[3.3.0]octan 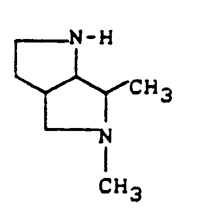 Man hydriert 10,8 g (46,9 mmol) 2-Benzyl-7,8-dimethyl-2,7-diazabicyclo[3.3.0]octan in 200 ml Ethanol an 2,5 g Palladium-Aktivkohle bei 100°C und 100 bar. Man saugt den Katalysator ab, engt das Filtrat ein und destilliert den Rückstand.
   Ausbeute: 4,3 g (65,4 % der Theorie)
   Siedepunkt: 60-62°C/6 mbar

### Beispiel 22

### 4-Methyl-2,7-diazabicyclo[3.3.0]octan

a) N-(2-Buten-1-yl)-N-(2,2-dimethoxyethyl)-amin 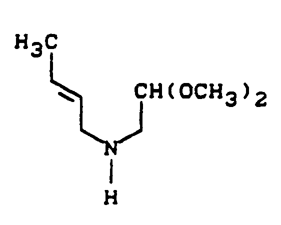 Man legt 200 g Molsieb in 1.000 ml Ethanol vor und setzt 105 g (1 mol) Aminoacetaldehyddimethylacetal und 70 g (1 mol) Crotonaldehyd hinzu. Man läßt über Nacht bei Raumtemperatur stehen, dekantiert vom Molsieb ab, kühlt auf 0°C und setzt 40 g Natriumborhydrid in 1 g-Portionen hinzu. Anschließend rührt man über Nacht bei Raumtemperatur, engt ein, nimmt in 500 ml Wasser auf und versetzt mit Kaliumcarbonat, bis sich eine organische Phase abscheidet. Man extrahiert mit Chloroform, trocknet über Kaliumcarbonat, engt ein und destilliert.
   Ausbeute: 69,5 g (41,5 % der Theorie)
   Siedepunkt: 85°C/12 mbar
b) N-(2-Buten-1-yl)-N-(2,2-dimethoxyethyl)-carbamidsäureethylester 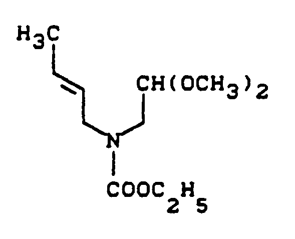 Man löst 69 g (0,41 mol) N-(2-Buten-1-yl)-N-(2,2-dimethoxyethyl)-amin in 200 ml Toluol, setzt 30 ml 45 %ige Natronlauge hinzu und tropft unter Eiskühlung 43 g (0,44 mol) Chlorameisensäureethylester hinzu. Man rührt noch drei Stunden bei Raumtemperatur, trennt die wäßrige Phase ab und extrahiert sie mit 100 ml Toluol. Man trocknet über Kaliumcarbonat, engt ein und destilliert.
   Ausbeute: 92 g (94 % der Theorie)
   Siedepunkt: 72°C/0,08 mbar
   Man löst 90 g (0,5 mol) N-(2,2-Dimethoxyethyl)carbamidsäureethylester in 500 ml Toluol, setzt 100 g gepulvertes Kaliumhydroxid und 1,5 g Triethylbenzylammoniumchlorid hinzu und tropft 80 g (0,6 mol) Crotylbromid (Isomerengemisch) hinzu. Man rührt über Nacht bei Raumtemperatur, löst die Salze in Wasser, trennt die wäßrige Phase ab und extrahiert sie einmal mit 100 ml Toluol. Man trocknet über Kaliumcarbonat, engt ein und destilliert.
   Ausbeute: 112 g (96,8 % der Theorie)
   Siedepunkt: 65°C/0,1 mbar
c) N-(2-Buten-1-yl)-N-(2-oxoethyl)-carbamidsäureethylester 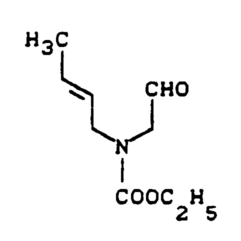 Man erhitzt 111 g (0,48 mol) N-(2-Buten-1-yl)-N-(2,2-dimethoxyethyl)-carbamidsäureethylester mit 50 g Ameisensäure in 950 ml Wasser drei Stunden unter Rückfluß. Man sättigt mit Kochsalz und extrahiert dreimal mit je 200 ml Methylenchlorid. Die organischen Phasen werden mit Natriumhydrogencarbonatlösung neutral gewaschen, über Magnesiumsulfat getrocknet, eingeengt und destilliert.
   Ausbeute: 77 g (86,6 % der Theorie)
   Siedepunkt: 94 bis 100°C/0,15 mbar
d) 2-Benzyl-4-methyl-2,7-diazabicyclo[3.3.0]octan-7-carbonsäureethylester 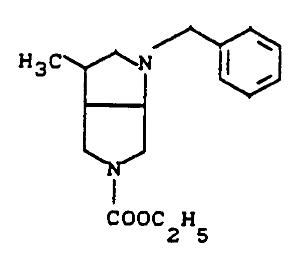 Man erhitzt 18,5 g (0,1 mol) N-(2-Buten-1-yl)-N-(2-oxoethyl)-carbamidsäureethylester mit 16,5 g (0,1 mol) N-Benzylglycin in 300 ml Toluol über Nacht am Wasserabscheider unter Rückfluß. Man engt ein und destilliert.
   Ausbeute: 10 g (25 % der Theorie)
   Siedepunkt: 135 bis 142°C/0,1 mbar
   Das Produkt ist gaschromatographisch 76 %ig.
e) 2-Benzyl-4-methyl-2,7-diazabicyclo[3.3.0]octan 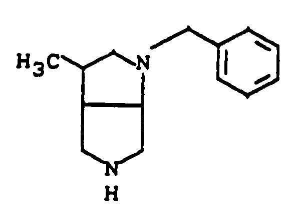 Man erhitzt 10 g (26,3 mmol) 2-Benzyl-4-methyl-2,7-diazabicyclo[3.3.0]octan-7-carbonsäureethylester mit 100 ml konzentrierter Salzsäure über Nacht unter Rückfluß. Man engt ein, nimmt in 20 ml Wasser auf, stellt mit Kaliumcarbonat alkalisch, extrahiert fünfmal mit je 50 ml Chloroform, trocknet über Kaliumcarbonat, engt ein und destilliert.
   Ausbeute: 4,6 g (81 % der Theorie)
   Siedepunkt: 87 bis 95°C/0,13 mbar
   Das Produkt ist gaschromatographisch 76 %ig.
f) 4-Methyl-2,7-diazabicyclo[3.3.0]octan 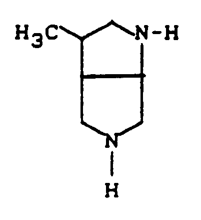 Man hydriert 4,1 g (19 mmol) 2-Benzyl-4-methyl-2,7-diazabicyclo[3.3.0]octan in 80 ml Methanol an 1 g Palladium-Aktivkohle (10 % Pd) bei 100°C und 100 bar. Der Katalysator wird abgesaugt, das Filtrat eingeengt und der Rückstand destilliert.
   Ausbeute: 1,2 g (50 % der Theorie)
   Siedepunkt: 76°C/8 mbar

### Beispiel 23

### 5-Fluormethyl-2-methyl-2,7-diazabicyclo[3.3.0]octan

a) N-(2-Fluormethylallyl)-N-(2,2-dimethoxyethyl)carbamidsäureethylester 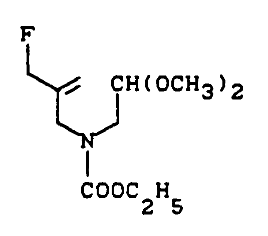 Man legt 8 g (0,26 mol) Natriumhydrid (80 %ig) in 200 ml Toluol vor und tropft bei 90°C 35,8 g (0,2 mol) N-(2,2-Dimethoxyethyl)-carbamidsäureethylester hinzu. Anschließend rührt man eine Stunde bei 90°C und tropft dann 32,6 g (0,3 mol) 1-Chlor-2-fluormethylpropen-2 hinzu. Man rührt über Nacht bei 90°C, löst Salze in Wasser, trennt die wäßrige Phase ab und extrahiert sie mit Toluol. Die organischen Phasen werden über Kaliumcarbonat getrocknet, eingeengt und destilliert.
   Ausbeute: 28,2 g (56,6 % der Theorie)
   Siedepunkt: 71 bis 79°C/0,07 mbar
b) N-(2-Fluormethylallyl)-N-(2-oxoethyl)-carbamidsäureethylester 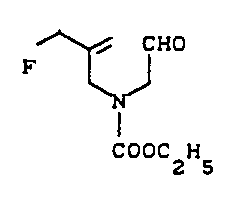 Man erhitzt 25 g (0,1 mol) N-(2-Fluormethylallyl)-N-(2,2-dimethoxyethyl-carbamidsäureethylester mit 5 g Ameisensäure in 100 ml Wasser zwei Stunden unter Rückfluß. Man sättigt mit Kochsalz, extrahiert mit Methylenchlorid und wäscht die organischen Phasen mit Natriumhydrogencarbonatlösung neutral. Man trocknet über Magnesiumsulfat, engt ein und destilliert.
   Ausbeute: 18,5 g (87 % der Theorie)
   Siedepunkt: 84°C/0,18 mbar
c) 5-Fluormethyl-2-methyl-2,7-diazabicyclo[3.3.0]octan-7-carbonsäureethylester 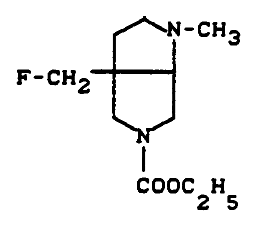 Man erhitzt 9,1 g (43 mmol) N-(2-Fluormethylallyl)-N-(2-oxoethyl)-carbamidsäureethylester mit 3,9 g (43 mmol) gepulvertem Sarkosin in 170 ml Toluol über Nacht am Wasserabscheider unter Rückfluß. Man engt ein und destilliert den Rückstand.
   Ausbeute: 7,5 g (75,8 % der Theorie)
   Siedepunkt: 80 bis 100°C/0,25 bis 0,35 mbar
d) 5-Fluormethyl-2-methyl-2,7-diazabicyclo[3.3.0]octan 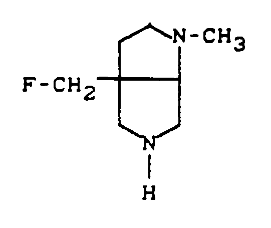 Man erhitzt 7,1 g (26 mmol) 5-Fluormethyl-2-methyl-2,7-diazabicyclo[3.3.0]octan-7-carbonsäureethylester in 100 ml konzentrierter Salzsäure über Nacht unter Rückfluß. Man engt ein, nimmt in 20 ml Wasser auf, stellt mit Kaliumcarbonat alkalisch, extrahiert zehnmal mit je 50 ml Chloroform, trocknet über Kaliumcarbonat, engt ein und destilliert.
   Ausbeute: 0,8 g (20 % der Theorie)
   Siedepunkt: 34°C/0,07 mbar

### Beispiel 24

### 5-Fluor-7-methyl-2,7-diazabicyclo[3.3.0]octan

a) N-(2,2-Dimethoxyethyl)-N-(2-fluorallyl)-carbamidsäureethylester 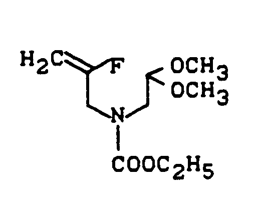 Man legt 11,6 g (65,5 mmol) N-(2,2-Dimethoxyethyl)-carbamidsäureethylester, 15 g gepulvertes Kaliumhydroxid und 0,25 g Triethylbenzylammoniumchlorid in 65 ml Toluol vor und tropft 10 g (72 mmol) 2-Fluorallylbromid bei Raumtemperatur hinzu. Man rührt über Nacht bei Raumtemperatur, setzt 100 ml Wasser hinzu, trennt die wäßrige Phase ab und extrahiert sie mit 30 ml Toluol. Die organischen Lösungen werden über Magnesiumsulfat getrocknet, eingeengt und der Rückstand destilliert.
   Ausbeute: 14,1 g (91,5 % der Theorie)
   Siedepunkt: 72°C/0,3 mbar
b) N-(2-Fluorallyl)-N-(2-oxoethyl)-carbamidsäureethylester 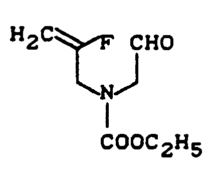 Man erhitzt 14,1 g (60 mmol) N-(2,2-Dimethoxyethyl)-N-(2-fluorallyl)-carbamidsäureethylester mit 6,3 ml Ameisensäure in 120 ml Wasser drei Stunden unter Rückfluß. Man sättigt die Lösung mit Kochsalz, extrahiert mehrfach mit Methylenchlorid, wäscht die organischen Lösungen mit gesättigter Natriumhydrogencarbonatlösung, trocknet über Magnesiumsulfat, engt ein und destilliert den Rückstand.
   Ausbeute: 9,8 g (86 % der Theorie)
   Siedepunkt: 80°C/0,25 mbar
c) 2-Benzyl-5-fluor-2,7-diazabicyclo[3,3,0]octan-7-carbonsäureethylester 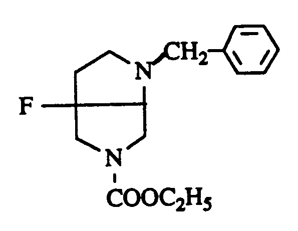 Man erhitzt 20,8 g (0,11 mol) N-(2-Fluorallyl)-N-(2-oxoethyl)-carbamidsäureethylester mit 19 g (0,115 mol) N-Benzylglycin in 300 ml Toluol bis zum Ende der CO₂-Entwicklung unter Rückfluß. Man engt ein und destilliert.
   Ausbeute: 16,4 g (44,8 % der Theorie)
   Siedepunkt: 148-152°C/0,1 mbar
   Das Produkt ist gaschromatographisch 88 %ig
d) 2-Benzyl-5-fluor-7-methyl-2,7-diazabicyclo[3.3.0]octan 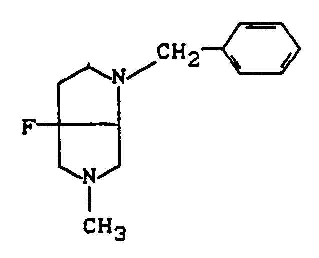 Zu 4,3 g (0,11 mol) Lithiumaluminiumhydrid in 125 ml absolutem Tetrahydrofuran tropft man eine Lösung von 16,4 g (49,4 mmol, 88 %ig) 2-Benzyl-5-fluor-2,7-diazabicyclo[3.3.0]octan-7-carbonsäureethylester in 25 ml absolutem Tetrahydrofuran und erhitzt anschließend über Nacht unter Rückfluß. Man zersetzt nacheinander mit je 4,5 ml Wasser, 15 %iger Kalilauge und Wasser, saugt die anorganischen Salze ab und kocht sie dreimal mit je 50 ml Tetrahdrofuran aus. Die organischen Lösungen werden eingeengt und der Rückstand destilliert.
   Ausbeute: 11 g (88 % der Theorie)
   Siedepunkt: 98-108°C/0,08 mbar
   Das Produkt ist gaschromatographisch 93 %ig.
e) 5-Fluor-7-methyl-2,7-diazabicyclo[3,3,0]octan 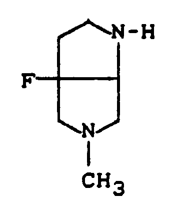 Man hydriert 11 g (43,7 mmol, 93 %ig) 2-Benzyl-5-fluor-7-methyl-2,7-diazabicyclo[3,3,0]octan in 100 ml Ethanol an 2 g Palladium-Aktivkohle (10 % Pd) bei 100°C und 100 bar. Man suagt den Katalysator ab, engt das Filtrat ein und destilliert den Rückstand.
   Ausbeute: 4,4 g (69,8 % der Theorie)
   Siedepunkt: 85-90°C/25 mbar

### Beispiel 25

### 6-Methyl-2,7-diazabicyclo[3,3,0]octan-7-carbonsäureethylester

a) N-(1-Buten-3-yl)-N-(2,2-dimethyloxyethyl)-carbamidsäureethylester 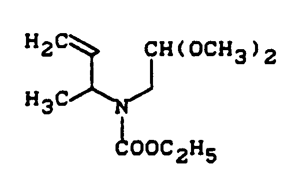 Zu 35,5 g (0,2 mol) N-(2,2-Dimethoxyethyl)-carbamidsäureethylester und 26 g gepulvertem Kaliumhydroxid in 400 ml Dimethylformamid gibt man 22 g (0,24 mol) 3-Chlor-1-buten und erwärmt über Nacht auf 40°C. Man löst die Salze mit Wasser extrahirt mehrfach mit Methylenchlorid. Die organischen Extrakte werden über Kaliumcarbonat getrocknet, eingeengt und destilliert.
   Ausbeute: 28,5 g (61,6 % der Theorie)
   Siedepunkt: 60°C/0,08 mbar
b) N-(1-Buten-3-yl)-N-(2-oxoethyl)-carbamidsäureethylester 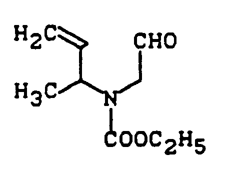 Man erhitzt 28,3 g (0,122 mol)N-(1-Buten-3-yl)-N-(2,2-dimethoxyethyl)-carbamidsäureethylester mit 65 ml Ameisensäure eine Stunde auf 100°C. Man gießt auf 200 g Eis, extrahiert mit Methylenchlorid, wäscht die organischen Extrakte mit gesättigter Natriumhydrogencarbonatlösung, trocknet über Magnesiumsulfat, engt ein und destilliert.
   Ausbeute: 11,6 g (51,3 % der Theorie)
   Siedepunkt: 62-65°C/0,03 mbar
c) 2-Benzyl-6-methyl-2,7-diazabicyclo[3.3.0]octan-7-carbonsäureethylester 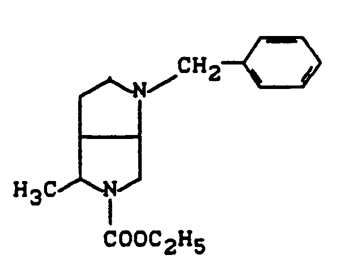 Man erhitzt 11,6 g (62,6 mmol) N-(1-Buten-3-yl)-N-(2-oxoethyl)-carbamidsäureethylester und 10,4 g (62,6 mmol) N-Benzylglycin in 170 ml Toluol über Nacht im Wasserabscheider unter Rückfluß. Man engt ein und destilliert.
   Ausbeute: 13,7 g (75,9 % der Theorie)
   Siedepunkt: 140-153°C/0,1 mbar.
d) 6-Methyl-2,7-diazabicyclo[3,3,0]octan-7-carbonsäureethylester 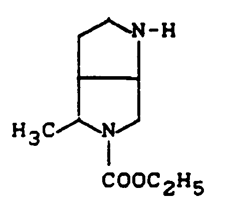 Man hydriert 13 g (44,9 mmol) 2-Benzyl-6-methyl-2,7-diazabicyclo[3,3,0]octan-7-carbonsäureethylester in 150 ml Ethanol an 2 g Palladium-Aktivkohle (10 % Pd) bei 100°C und 100 bar. Der Katalysator wird abfiltriert, das Filtrat eingeengt und destilliert.
   Ausbeute: 6,8 g (76,4 % der Theorie)
   Siedepunkt: 81°C/0,09 mbar

### Beispiel 26

### 2,7-Diazabicyclo[3.3.0]octan-3,7-dicarbonsäurediethylester

a) 2-Benzyl-2,7-diazabicyclo[3.3.0]octan-3,7-dicarbonsäurediethylester 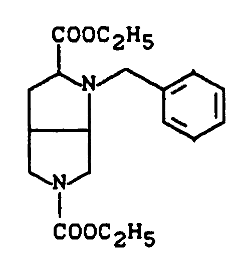 Man erhitzt 50 g (0,25 mol) N-Benzylglycinethylester in 1 l Toluol im Wasserabscheider unter Rückfluß und tropft innerhalb von zwei Stunden 43 g (0,25 mol) N-Allyl-N-(2-oxoethyl)-carbamidsäureethylester hinzu. Man erhitzt unter Rückfluß bis kein Wasser mehr abgeschieden wird, engt ein und destilliert den Rückstand.
   Ausbeute: 82,1 g (94,8 % der Theorie)
   Siedepunkt: 160-165°C/0,05 mbar
b) 2,7-Diazabicyclo[3,3,0]octan-3,7-dicarbonsäurediethylester 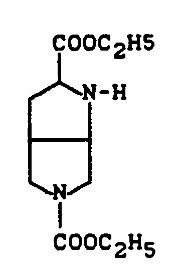 Man hydriert 96,5 g (0,279 mol) 2-Benzyl-2,7-diazabicyclo[3.3.0]octan-3,7-dicarbonsäurediethylester in 1 l Ethanol an 5 g Palladium-Aktivkohle (10 % Pd) bei 100°C und 100 bar. Man saugt ab, engt ein und destilliert.
   Ausbeute: 63,3 g (84,6 % der Theorie)
   Siedepunkt: 137-140°C/0,18-0,2 mbar

### Beispiel 27 (Endprodukt)

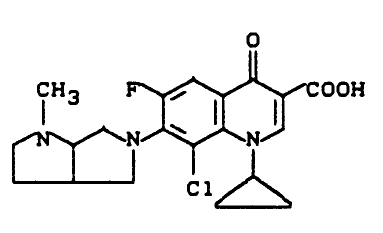

3 g (10 mmol) 8-Chlor-1-cyclopropyl-6,7-difluor-1,4-dihydro-4-oxo-3-chinolincarbonsäure werden in einer Mischung aus 30 ml Acetonitril und 15 ml Dimethylformamid mit 1,7 g (15 mmol) 1,4-Diazabicyclo[2.2.2]octan und 1,4 g (11 mmol) 2-Methyl-2,7-diazabicyclo[3.3.0]octan versetzt und 1 Stunde unter Rückfluß erhitzt. Die Mischung wird eingedampft, der Rückstand mit Wasser verrührt und der ungelöste Niederschlag abgesaugt, mit Wasser gewaschen und bei 120°C im Vakuum getrocknet.
Ausbeute: 2,4 g (59 % der Theorie) 8-Chlor-1-cyclopropyl-6-fluor-1,4-dihydro-7-(2-methyl-2,7-diazabicyclo[3.3.0]oct-7-yl)-4-oxo-3-chinolincarbonsäure, Schmelzpunkt: 208-213°C (unter Zersetzung) (aus Glykolmonomethylether).

## Patentansprüche (Patentansprüche für folgende(n) Vertragsstaat(en): AT, BE, CH, DE, DK, FR, GB, GR, IT, LI, LU, NL, SE)

1. Verfahren zur Herstellung von 2,7-Diazabicyclo[3.3.0]octanen der Formel (I), 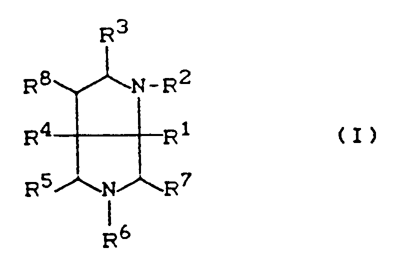 wobei
R¹, R³, R⁴, R⁵, R⁷, R⁸ gleich oder verschieden sein können und jeweils H, C₁-C₅-Alkyl (gegebenenfalls substituiert mit Halogen, Hydroxy oder C₁-C₃-Alkoxy), C₁-C₃-Alkoxycarbonyl, C₆-C₁₂-Aryl, bevorzugt H, C₁-C₃-Alkyl, C₁-C₃-Alkoxycabonyl, Phenyl, besonders bevorzugt H und Methyl bedeuten,
R⁴ zusätzlich Halogen, bevorzugt Fluor, Chlor oder Brom, besonders bevorzugt Chlor und Brom bedeutet,
R² und R⁶ gleich oder verschieden sein können und H, C₁-C₆-Alkyl, Benzyl, C₆-C₁₂-Aryl, C₁-C₃-Alkanoyl, Benzoyl, C₁-C₅-Alkoxycarbonyl bedeuten und gegebenenfalls
R² und R³ zusammen eine Brücke der Struktur (CH₂)ₙ, CH₂-CHOH-CH₂, CH₂-S-CH₂, C(CH₃)₂-S-CH₂ bedeuten, worin
n für 2-4 steht,
dadurch gekennzeichnet, daß man ungesättigte Carbonylverbindungen der Formel (II) mit Aminosäurederivaten der Formel (III) in einer intramolekularen 1,3-dipolaren Cycloaddition, 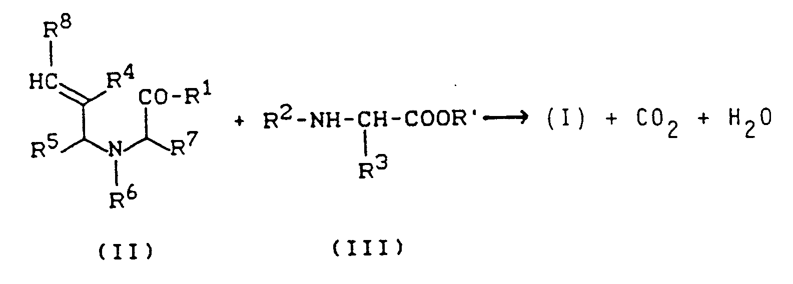 worin
R' für H oder C₁-C₃-Alkyl steht und worin R¹-R⁸ die oben angegebene Bedeutung haben, umsetzt und anschließend die Substituenten R² oder R⁶, soweit sie eine Schutzgruppenfunktion haben, abspaltet.

2. Verbindungen der Formel (II) 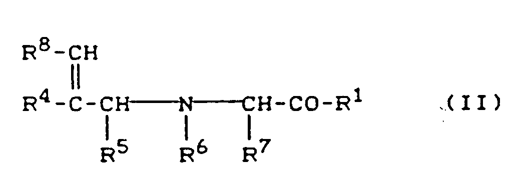 in denen
R¹, R⁴, R⁵, R⁷, R⁸ gleich oder verschieden sein können und jeweils H, C₁-C₅-Alkyl (gegebenenfalls substituiert mit Halogen, Hydroxy oder C₁-C₃-Alkoxy), C₁-C₃-Alkoxycarbonyl, C₆-C₁₂-Aryl, bevorzugt H, C₁-C₃-Alkyl, C₁-C₃-Alkoxycabonyl, Phenyl, besonders bevorzugt H und Methyl bedeuten,
R⁴ zusätzlich Halogen, bevorzugt Fluor, Chlor oder Brom, besonders bevorzugt Chlor und Brom bedeutet,
R⁶ H, C₁-C₆-Alkyl, Benzyl, C₆-C₁₂-Aryl, C₁-C₃-Alkanoyl, Benzoyl, C₁-C₅-Alkoxycarbonyl, bevorzugt H, C₁-C₃-Alkyl, Benzyl, C₆-C₁₂-Aryl, C₁-C₂-Alkanoyl, Benzoyl, C₁-C₄-Alkoxycarbonyl, besonders bevorzugt H, Methyl, Phenyl, Acetyl oder C₂-C₄-Alkoxycarbonyl bedeutet.

## Patentansprüche (Patentansprüche für folgende(n) Vertragsstaat(en): ES)

1. Verfahren zur Herstellung von 2,7-Diazabicyclo[3.3.0]octanen der Formel (I), 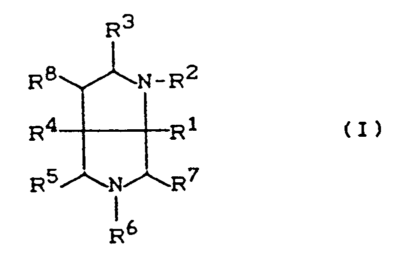 wobei
R¹, R³, R⁴, R⁵, R⁷, R⁸ gleich oder verschieden sein können und jeweils H, C₁-C₅-Alkyl (gegebenenfalls substituiert mit Halogen, Hydroxy oder C₁-C₃-Alkoxy), C₁-C₃-Alkoxycarbonyl, C₆-C₁₂-Aryl, bevorzugt H, C₁-C₃-Alkyl, C₁-C₃-Alkoxycabonyl, Phenyl, besonders bevorzugt H und Methyl bedeuten,
R⁴ zusätzlich Halogen, bevorzugt Fluor, Chlor oder Brom, besonders bevorzugt Chlor und Brom bedeutet,
R² und R⁶ gleich oder verschieden sein können und H, C₁-C₆-Alkyl, Benzyl, C₆-C₁₂-Aryl, C₁-C₃-Alkanoyl, Benzoyl, C₁-C₅-Alkoxycarbonyl bedeuten und gegebenenfalls
R² und R³ zusammen eine Brücke der Struktur (CH₂)ₙ, CH₂-CHOH-CH₂, CH₂-S-CH₂, C(CH₃)₂-S-CH₂ bedeuten, worin
n für 2-4 steht,
dadurch gekennzeichnet, daß man ungesättigte Carbonylverbindungen der Formel (II) mit Aminosäurederivaten der Formel (III) in einer intramolekularen 1,3-dipolaren Cycloaddition, 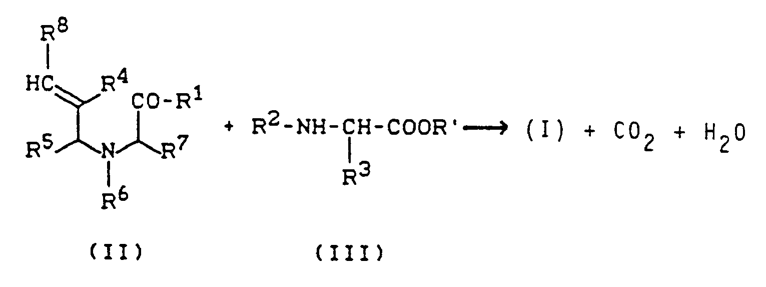 worin
R' für H oder C₁-C₃-Alkyl steht und worin R¹-R⁸ die oben angegebene Bedeutung haben, umsetzt und anschließend die Substituenten R² oder R⁶, soweit sie eine Schutzgruppenfunktion haben, abspaltet.

## Claims (Claims for the following Contracting State(s): AT, BE, CH, DE, DK, FR, GB, GR, IT, LI, LU, NL, SE)

1. Process for the preparation of 2,7-diazabicyclo[3.3.0]octanes of the formula (I) 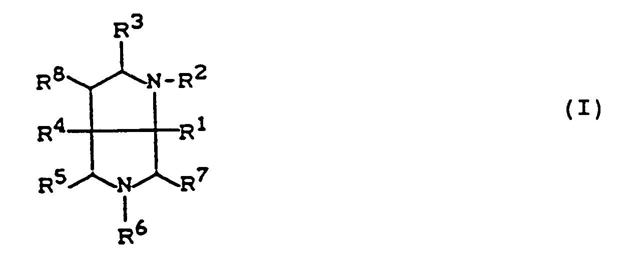 where
R¹, R³, R⁴, R⁵, R⁷ and R⁸ may be identical or different and in each case denote H, C₁-C₅-alkyl (optionally substituted by halogen, hydroxyl or C₁-C₃-alkoxy), C₁-C₃-alkoxycarbonyl or C₆-C₁₂-aryl, preferably H, C₁-C₃-alkyl, C₁-C₃-alkoxycarbonyl or phenyl, particularly preferably H and methyl,
R⁴ additionally denotes halogen, preferably fluorine, chlorine or bromine, particularly preferably chlorine and bromeine,
R² and R⁶ may be identical or different and denote H, C₁-C₆-alkyl, benzyl, C₆-C₁₂-aryl, C₁-C₃-alkanoyl, benzoyl or C₁-C₅-alkoxycarbonyl and, if appropriate,
R² and R³ together denote a bridge of the structure (CH₂)ₙ, CH₂-CHOH-CH₂, CH₂-S-CH₂ or C(CH₃)₂-S-CH₂,
in which
n represents 2-4,
characterized in that unsaturated carbonyl compounds of the formula (II) are reacted with amino acid derivatives of the formula (III) in an intramolecular 1,3-dipolar cycloaddition 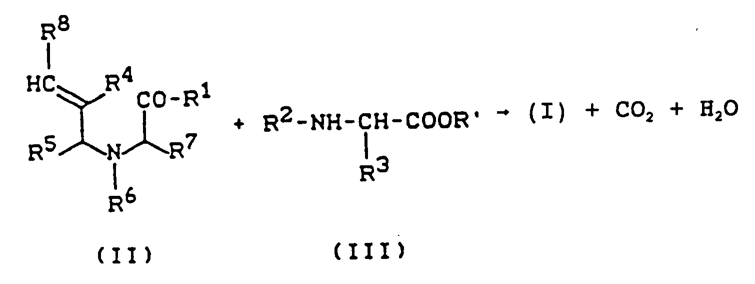 in which
R' represents H or C₁-C₃-alkyl and in which
R¹-R⁸ have the abovementioned meaning, and the substituents R² or R⁶, in so far as they have a protective group function, are then removed.

2. Compounds of the formula (II) 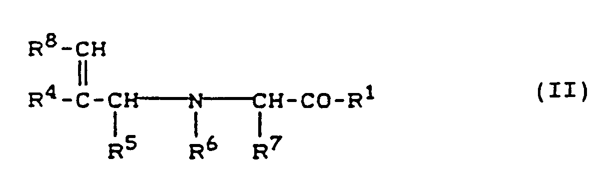 in which
R¹, R⁴, R⁵, R⁷ and R⁸ may be identical or different and in each case denote H, C₁-C₅-alkyl (optionally substituted by halogen, hydroxyl or C₁-C₃-alkoxy), C₁-C₃-alkoxycarbonyl or C₆-C₁₂-aryl, preferably H, C₁-C₃-alkyl, C₁-C₃-alkoxycarbonyl or phenyl, particularly preferably H and methyl,
R⁴ additionally denotes halogen, preferably florine, chlorine or bromine, particularly preferably chlorine and bromine,
R⁶ denotes H, C₁-C₆-alkyl, benzyl, C₆-C₁₂-aryl, C₁-C₃-alkanoyl, benzoyl or C₁-C₅-alkoxycarbonyl, preferably H, C₁-C₃-alkyl, benzyl, C₆-C₁₂-aryl, C₁-C₂-alkanoyl, benzoyl or C₁-C₄-alkoxycarbonyl, particularly preferably H, methyl, phenyl, acetyl or C₂-C₄-alkoxycarbonyl.

## Claims (Claims for the following Contracting State(s): ES)

1. Process for the preparation of 2,7-diazabicyclo[3.3.0]octanes of the formula (I) 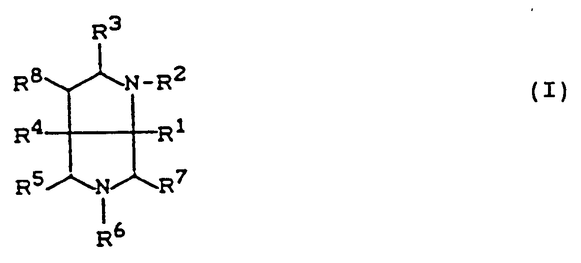 where
R¹, R³, R⁴, R⁵, R⁷ and R⁸ may be identical or different and in each case denote H, C₁-C₅-alkyl (optionally substituted by halogen, hydroxyl or C₁-C₃-alkoxy), C₁-C₃-alkoxycarbonyl or C₆-C₁₂-aryl, preferably H, C₁-C₃-alkyl, C₁-C₃-alkoxycarbonyl or phenyl, particularly preferably H and methyl,
R⁴ additionally denotes halogen, preferably fluorine, chlorine or bromine, particularly preferably chlorine and bromeine,
R² and R⁶ may be identical or different and denote H, C₁-C₆-alkyl, benzyl, C₆-C₁₂-aryl, C₁-C₃-alkanoyl, benzoyl or C₁-C₅-alkoxycarbonyl and, if appropriate,
R² and R³ together denote a bridge of the structure (CH₂)ₙ, CH₂-CHOH-CH₂, CH₂-S-CH₂ or C(CH₃)₂-S-CH₂,
in which
n represents 2-4,
characterized in that unsaturated carbonyl compounds of the formula (II) are reacted with amino acid derivatives of the formula (III) in an intramolecular 1,3-dipolar cycloaddition 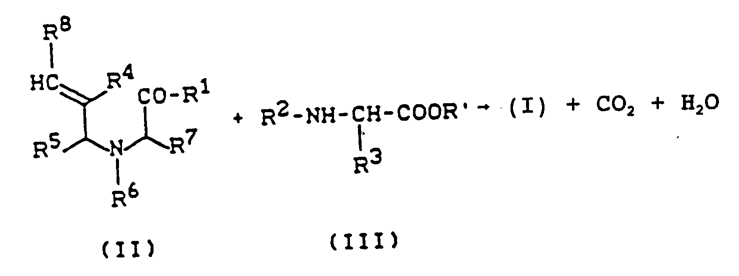 in which
R' represents H or C₁-C₃-alkyl and in which
R¹-R⁸ have the abovementioned meaning, and the substituents R² or R⁶, in so far as they have a protective group function, are then removed.

## Revendications (Revendications pour l'(les) Etat(s) contractant(s) suivant(s): AT, BE, CH, DE, DK, FR, GB, GR, IT, LI, LU, NL, SE)

1. Procédé pour la préparation de 2,7-diazabicyclo[3.3.0]octanes de formule (I) 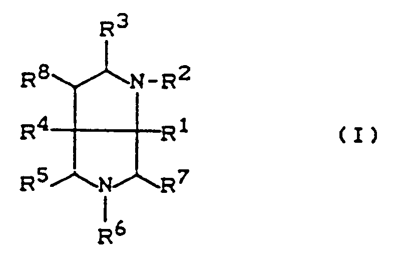 dans laquelle
R¹, R³, R⁴, R⁵, R⁷, R⁸ peuvent être identiques ou différents et représentent respectivement H, un groupe alkyle en C₁-C₅ (portant éventuellement un ou plusieurs substituants halogéno, hydroxyle ou alcoxy en C₁-C₃), un groupe alcoxy(en C₁-C₃)carbonyle, un groupe aryle en C₆-C₁₂; de préférence H, un groupe alkyle en C₁-C₃, un groupe alcoxy(en C₁-C₃)carbonyle, un groupe phényle; de manière particulièrement préférée H et un groupe méthyle,
R⁴ représente, en outre, un atome d'halogène; de préférence un atome de fluor, un atome de chlore ou un atome de brome; de manière particulièrement préférée un atome de chlore et un atome de brome,
R² et R⁶ peuvent être identiques ou différents et représentent H, un groupe alkyle en C₁-C₆, un groupe benzyle, un groupe aryle en C₆-C₁₂, un groupe alcanoyle en C₁-C₃, un groupe benzoyle, un groupe alcoxy(en C₁-C₅) carbonyle et éventuellement
R² et R³ ensemble représentent un pont de structure (CH₂)ₙ, CH₂-CHOH-CH₂, CH₂-S-CH₂, C(CH₃)₂-S-CH₂, où
n représente 2-4,
caractérisé en ce qu'on fait réagir des composés carbonyle insaturés de formule (II) avec des dérivés d'aminoacides de formule (III) dans une cycloaddition 1,3-dipolaire intramoléculaire 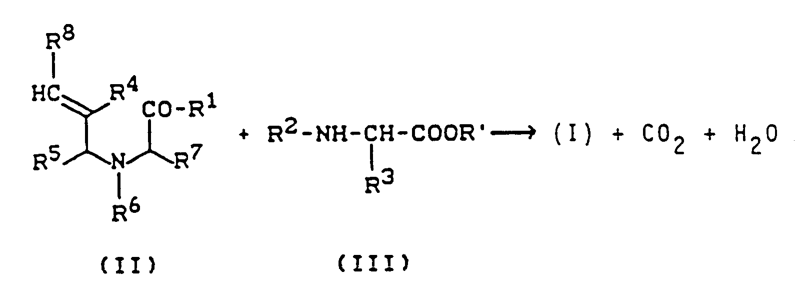 dans laquelle
R' représente H ou un groupe alkyle en C₁-C₃ et
dans laquelle R¹-R⁸ ont la signification indiquée ci-dessus, et ensuite, on sépare les substituants R² ou R⁶ pour autant qu'ils possèdent une fonction de groupes de protection.

2. Composés de formule (II) 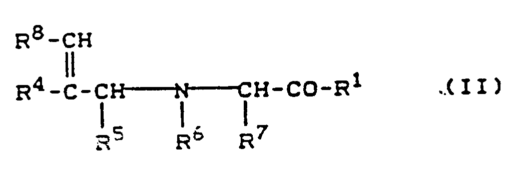 dans lesquels
R¹, R⁴, R⁵, R⁷, R⁸ peuvent être identiques ou différents et représentent respectivement H, un groupe alkyle en C₁-C₅, (portant éventuellement un ou plusieurs substituants halogéno, hydroxyle ou alcoxy en C₁-C₃), un groupe alcoxy(en C₁-C₃)carbonyle, un groupe aryle en C₆-C₁₂; de préférence H, un groupe alkyle en C₁-C₃, un groupe alcoxy(en C₁-C₃)carbonyle, un groupe phényle; de manière particulièrement préférée H et un groupe méthyle,
R⁴ représente, en outre, un atome d'halogène; de préférence un atome de fluor, un atome de chlore ou un atome de brome; de manière particulièrement préférée un atome de chlore et un atome de brome,
R⁶ représente H, un groupe alkyle en C₁-C₆, un groupe benzyle, un groupe aryle en C₆-C₁₂, un groupe alcanoyle en C₁-C₃, un groupe benzoyle, un groupe alcoxy(en C₁-C₅)carbonyle; de préférence H, un groupe alkyle en C₁-C₃, un groupe benzyle, un groupe aryle en C₆-C₁₂, un groupe alcanoyle en C₁-C₂, un groupe benzoyle, un groupe alcoxy(en C₁-C₄)carbonyle; de manière particulièrement préférée H, un groupe méthyle, un groupe phényle, un groupe acétyle ou encore un groupe alcoxy(en C₂-C₄)carbonyle.

## Revendications (Revendications pour l'(les) Etat(s) contractant(s) suivant(s): ES)

1. Procédé pour la préparation de 2,7-diazabicyclo[3.3.0]octanes de formule (I) 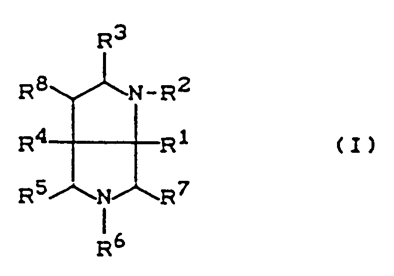 dans laquelle
R¹, R³, R⁴, R⁵, R⁷, R⁸ peuvent être identiques ou différents et représentent respectivement H, un groupe alkyle en C₁-C₅ (portant éventuellement un ou plusieurs substituants halogéno, hydroxyle ou alcoxy en C₁-C₃), un groupe alcoxy(en C₁-C₃)carbonyle, un groupe aryle en C₆-C₁₂; de préférence H, un groupe alkyle en C₁-C₃, un groupe alcoxy(en C₁-C₃)carbonyle, un groupe phényle; de manière particulièrement préférée H et un groupe méthyle,
R⁴ représente, en outre, un atome d'halogène; de préférence un atome de fluor, un atome de chlore ou un atome de brome; de manière particulièrement préférée un atome de chlore et un atome de brome,
R² et R⁶ peuvent être identiques ou différents et représentent H, un groupe alkyle en C₁-C₆, un groupe benzyle, un groupe aryle en C₆-C₁₂, un groupe alcanoyle en C₁-C₃, un groupe benzoyle, un groupe alcoxy(en C₁-C₅) carbonyle et éventuellement
R² et R³ ensemble représentent un pont de structure (CH₂)ₙ, CH₂-CHOH-CH₂, CH₂-S-CH₂, C(CH₃)₂-S-CH₂, où
n représente 2-4,
caractérisé en ce qu'on fait réagir des composés carbonyle insaturés de formule (II) avec des dérivés d'aminoacides de formule (III) dans une cycloaddition 1,3-dipolaire intramoléculaire 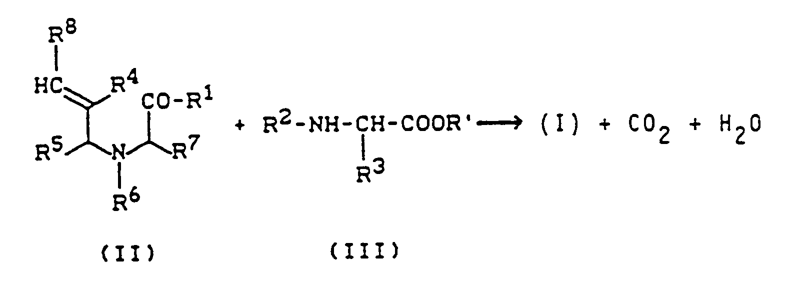 dans laquelle
R' représente H ou un groupe alkyle en C₁-C₃ et
dans laquelle R¹-R⁸ ont la signification indiquée ci-dessus, et ensuite, on sépare les substituants R² ou R⁶ pour autant qu'ils possèdent une fonction de groupes de protection.
